# EUROPÄISCHE PATENTSCHRIFT

(11) **EP 2 548 023 B1**
(45) Veröffentlichungstag und Bekanntmachung des Hinweises auf die Patenterteilung: **16.11.2016**
(21) Anmeldenummer: 11711029.6
(22) Anmeldetag: 14.03.2011
(51) Int. Cl.: G01N 33/52, G01N 33/53, G01N 33/543, B01L 3/00

(54) **VORRICHTUNG UND VERFAHREN ZUR MANIPULATION ODER UNTERSUCHUNG EINER FLÜSSIGEN PROBE**
DEVICE AND METHOD FOR MANIPULATING OR EXAMINING A LIQUID SAMPLE
DISPOSITIF ET PROCÉDÉ DE MANIPULATION OU D'ANALYSE D'UN ÉCHANTILLON LIQUIDE

(30) Priorität: 15.03.2010 EP 10002685
(43) Veröffentlichungstag der Anmeldung: 23.01.2013
(73) Patentinhaber: BOEHRINGER INGELHEIM INTERNATIONAL GMBH, 55216 Ingelheim am Rhein (DE)
(72) Erfinder: RODENFELS, Tobias, 55216 Ingelheim am Rhein (DE); BLANKENSTEIN, Gert, 55216 Ingelheim am Rhein (DE); OSTERLOH, Dirk, 55216 Ingelheim am Rhein (DE)
(74) Vertreter: Simon, Elke Anna Maria
(86) Internationale Anmeldenummer: PCT/EP2011/001254
(87) Internationale Veröffentlichungsnummer: WO 2011/113569

(56) Entgegenhaltungen:
- WO-A2-2007/089564
- DE-A1-102004 062 534
- US-A- 5 439 650
- Sawetzki, Tobias: "Mikropartikel als aktive Komponenten mikrofluidischer Operationen", E-Lib Universität Stuttgart Dissertation, 28. Januar 2010 (2010-01-28), XP002590552, Gefunden im Internet: URL:http://elib.uni-stuttgart.de/opus/voll texte/2010/4899/ [gefunden am 2010-07-05]
- KARLE, M; MIWA, JUNICHI;ROTH, GÜNTER; HAEBERLE, STEFAN; ZENGERLE, ROLAND; VON STETTEN, FELIX: "Kontinuierlich arbeitende Mikrofluidik-Plattform zur Aufreinigung von Biomolekülen", VDE Verlag GmbH, PAPER 31, 12. Oktober 2009 (2009-10-12), Seiten 1-4, XP002590553, Berlin Offenbach ISBN: 978-3-8007-3183-1 Gefunden im Internet: URL:http://www.imtek.de/content/pdf/public /2009/kontinuierlich_arbeitende_mikrofluid ik-plattfo.pdf [gefunden am 2010-07-05]
- VDI/VDE/IT: "Integrierte mikrofluidische Diagnosesysteme - IMIKRID", , 31. Mai 2010 (2010-05-31), Seiten 1-2, XP002590554, Gefunden im Internet: URL:http://www.mstonline.de/foerderung/pro jektliste/printable_pdf?vb_nr=W3BIO057 [gefunden am 2010-07-06]
- Mandy F; Varro R; Recktenwald D: "Flow Cytometry"; "25" In: Tuan Vo-Dinh: "Biomedical Photonics Handbook", March 2003 (2003-03), Crc Pr Inc ISBN: 0-8493-1116-0 page 25-11, * pages 25-11, paragraph 25.8.2 *

## Beschreibung

Die vorliegende Erfindung betrifft eine Vorrichtung und ein Verfahren zur Manipulation und/oder Untersuchung einer flüssigen Probe gemäß dem Oberbegriff des Anspruchs 1, 3 oder 23.

Die vorliegende Erfindung befasst sich insbesondere mit der Diagnostik bei mikrofluidischen Proben, besonders bevorzugt bei so genannten Point-Off Care-Systemen (POC-Systemen). Insbesondere befasst sich die vorliegende Erfindung mit vorzugsweise miniaturisierten Immunoassays, also der Untersuchung von Proben unter Einsatz von Antikörpern. Besonders bevorzugt betrifft die vorliegende Erfindung so genannte Cartridge-Konzepte, also kleine, insbesondere kartenartige Vorrichtungen, zur Manipulation und/oder Untersuchung einer flüssigen Probe bzw. Realisierung von Immunoassays.

In "Kontinuierlich arbeitende Mikrofluidik-Plattform zur Aufreinigung von Biomolekülen" von Karle, Marc; Miwa, Junichi; Roth, Günter; Haeberle, Stefan; Zengerle, Roland; von Stetten, Felix; erschienen im VDE Verlag GmbH, Paper 31, 12. Oktober 2009, Seiten 1 bis 4, ISDN: 978-3-8007, 3183-1, wird eine kontinuierlich arbeitende Mikrofluidik-Plattform zur Aufreinigung von Biomolekülen offenbart. Die Plattform weist Kanäle und einen auf die Plattform wirkenden drehbaren Permanentmagneten auf. Magnetische Partikel, die sich entlang von Magnetlinien bewegen, transportieren aufzureinigende Biomoleküle durch die Kanäle. Durch das von außen angelegte Magnetfeld werden die magnetischen Partikel magnetophoretisch über eine Phasengrenze einer Laminarströmung von einem Puffer in einen nächsten überführt. Ein Übergang der Partikel von einer flüssigen Phase in einen Gasraum wird nicht offenbart, sondern nur von einer Flüssigkeit bzw. flüssigen Phase in eine andere.

Die US 2009/0227044 A1 offenbart eine Vorrichtung mit einem Mikrokanal, in dem magnetische, lumineszierende Nanopartikel als Träger für Antikörper und als interner Lumineszenzstandard dienen. Die Träger werden durch ein externes Magnetfeld, das durch Elektromagnete erzeugt wird, in Schwingung versetzt, um eine bessere Diffusion während eines Inkubationsschritts zu erreichen. Weiter werden die Elektromagnete verwendet, um die Partikel in dem Kanal für Waschschritte- und Lumineszenzmessungen zu halten.

Die WO 99/49319 A1 offenbart ein Mikrosystem zur Manipulation von magnetischen Partikeln. Die Partikel werden mit einem Reagenz oder Antikörper beschichtet und durch magnetische Kräfte in dem mikrofluidischen System bewegt. Unterschiedliche Partikel können mit unterschiedlichen Reagenzien versehen werden. Die Auswertung erfolgt magnetisch und/oder durch Fluoreszenzmessung.

Die US 2002/0064866 A1 offenbart fibröse, sphärische Partikel als Träger von Substanzen zur Durchführung von Tests. Hierzu werden Substanzen an die zerklüftete Oberfläche der Partikel gebunden bzw. in deren Oberfläche eingelagert. Die Partikel werden als Suspension aufgezogen und bei Bedarf über magnetische Kräfte festgehalten.

Die US 7,105,357 B1 offenbart ein Verfahren mit einer Pipette zur Erzeugung kleinster Tropfen, wobei Substanzen an magnetische Partikel gebunden werden und diese in der Pipette mittels Permanentmagneten festgehalten werden.

Die US 5,135,720 A offenbart ein Reaktionsgefäß mit einem kugelförmigen Träger, der einen magnetischen Kern und eine Beschichtung aufweist, auf der Proteine oder Peptide befestigt werden können. Der Träger wird über Elektromagnete und einen Regelmechanismus in Schwebe gehalten, um die Effizienz einer Edman-Reaktion mit auf der Oberfläche des Trägers befindlichen Substanzen zu verbessern.

Die US 5,439,650 A offenbart eine ähnliche Vorrichtung, wobei ein Teil des Reaktionsgefäßes mit dem schwebenden Träger und den diesem zugeordneten Elektromagneten verschiebbar ist.

Es hat sich gezeigt, dass die Vielzahl von kleinen magnetischen, im Wesentlichen kugelförmigen oder eine sonstige undefinierte Form aufweisenden Partikeln keine optimale Manipulation oder Untersuchung einer flüssigen Probe gestattet. Insbesondere ist keine optimale optische Detektion des Bindens von Analyten oder Komplexen an den Trägerpartikeln möglich.

Der vorliegenden Erfindung liegt die Aufgabe zugrunde, eine Vorrichtung und ein Verfahren zur Manipulation und/oder Untersuchung einer flüssigen Probe insbesondere in einem mikrofluidischen System bzw. Kanal anzugeben, wobei eine optimierte Manipulation oder Untersuchung und/oder eine verbesserte, vorzugsweise optische Detektion - insbesondere zum Nachweis einer Reaktion oder zur Bestimmung eines Analyten - ermöglicht wird bzw. werden und/oder wobei mehrere Untersuchungen parallel an einer Probe unter definierten Bedingungen und/oder räumlich getrennt durchgeführt werden können.

Die obige Aufgabe wird durch eine Vorrichtung gemäß Anspruch 1 oder 3 oder durch ein Verfahren gemäß Anspruch 23 gelöst. Vorteilhafte Weiterbildungen sind Gegenstand der Unteransprüche.

Die vorliegende Erfindung befasst sich mit der Manipulation und/oder Untersuchung einer flüssigen Probe insbesondere in einem mikrofluidischen Kanal oder System. Unter "mikrofluidisch" sind erfindungsgemäß Volumina von vorzugsweise weniger als 10 ml, besonders bevorzugt von weniger als 1 ml, und/oder Kanal- oder Flüssigkeitsquerschnitte (maximaler Durchmesser) von vorzugsweise weniger als 2 mm, besonders bevorzugt von weniger als 500 µm, zu verstehen.

Bei der vorliegenden Erfindung wird ein in einem vorzugsweise mikrofluidischen Kanal bzw. System beweglicher Träger eingesetzt, der mindestens einen Detektions- oder Bindungsbereich für einen Stoff aufweist. Dieser kann ein Analyt der Probe oder ein davon gebildeter Komplex oder davon abhängiges Reaktionsprodukt und/oder ein Reagenz sein, das mit der Probe, einem Analyten der Probe, einem Komplex davon oder dergleichen wechselwirkt oder bindet. Beispielsweise kann das Reagenz mit einem Komplex des Analyten oder mit einem vom Analyten abhängigen Reaktionsprodukt wechselwirken oder binden. Vorzugsweise ist das Reagenz selbst in oder auf dem Detektions- bzw. Bindungsbereich fixiert oder immobilisiert. Insbesondere kann der Detektions- bzw. Bindungsbereich einen immobilisierten Antikörper enthalten oder aufweisen, der mit einem Analyten der Probe oder einem den Analyten enthaltenden Komplex wechselwirkt, besonders bevorzugt bindet. In diesem Fall wird das Reagenz durch den Antikörper gebildet, der mit dem Analyten indirekt, nämlich mit einem den Analyten enthaltenden Komplex oder dergleichen, wechselwirkt. Es sind aber auch andere Wechselwirkungen oder Reaktionen realisierbar, beispielsweise eine Modifikation des Reagenzes oder Detektions- bzw. Bindungsbereichs oder ein Lösen des Reagenzes. Der Begriff "Wechselwirken" ist dementsprechend vorzugsweise in einem weiten Sinne bei der vorliegenden Erfindung zu verstehen.

Durch das Bewegen des Trägers über die Phasengrenze flüssig/gasförmig, also durch das Bewegen in den Gasraum oder durch den Gasraum, wird erreicht, dass möglichst wenig Flüssigkeit mit dem Träger transportiert wird. Vielmehr wird die Flüssigkeit in dem jeweiligen Flüssigkeitsraum zumindest weitestgehend zurückgehalten. Es erfolgt insbesondere quasi ein Abstreifen der Flüssigkeit beim Bewegen des Trägers durch die Phasegrenze in den Gasraum.

Gemäß einem Aspekt der vorliegenden Erfindung ist der Träger, insbesondere mittels einer Manipuliereinrichtung, aus der Probe heraus, insbesondere in einem Gasraum, bzw. über oder durch eine Phasengrenze flüssig/gasförmig oder umgekehrt bewegbar. Dies ist wiederum einer insbesondere optischen Detektion zuträglich.

Unter dem Begriff "Detektion" ist bei der vorliegenden Erfindung vorzugsweise das Erfassen einer Wechselwirkung, Modifikation oder Reaktion im Detektions- bzw. Bindungsbereich und/oder das Binden eines Stoffs, wie eines Analyten, Komplexes oder dergleichen, im Detektions- bzw. Bindungsbereich zu verstehen, um eine vorzugsweise qualitative und/oder quantitative Untersuchung der Probe, insbesondere eine qualitative und/oder quantitative Bestimmung mindestens eines Analyten der Probe, zu ermöglichen. Die Detektion kann insbesondere optisch, besonders bevorzugt durch Lumineszenz- oder Fluoreszenzmessung, erfolgen.

Gemäß einem weiteren Aspekt der vorliegenden Erfindung wird der Träger aus der Probe heraus in einen Gasraum des Systems zur Detektion eines im Detektions- oder Bindungsbereich gebundenen Stoffs bewegt. Dies gestattet eine optimierte, insbesondere optische Detektion, besonders bevorzugt mittels Lumineszenz- oder Fluoreszenzmessung.

Alternativ oder zusätzlich kann der Träger durch den Gasraum in eine von der Probe verschiedene Flüssigkeit oder umgekehrt bewegt werden. So können verschiedene Schritte, wie Inkubationsschritte, Wasch-Schritte oder dergleichen, insbesondere mit minimalen Flüssigkeitsvolumina realisiert werden.

Insbesondere bewirkt das Bewegen des Trägers über die Phasengrenze von flüssig zu gasförmig, dass keine oder nur sehr wenig Flüssigkeit von dem Träger mitgenommen bzw. mit transportiert wird. Daher wird durch das Bewegen in den Gasraum oder durch diesen hindurch insbesondere ein zumindest weitgehendes Separieren des Trägers von der in die flüssige Phase bildenden Flüssigkeit ermöglicht oder bewirkt.

Besonders bevorzugt soll bei der vorliegenden Erfindung unter Phasengrenze ein Übergang von flüssig zu gasförmig oder umgekehrt verstanden werden.

Gemäß einem Aspekt der vorliegenden Erfindung ist der Träger zumindest im Wesentlichen eben oder plattenförmig ausgebildet und/oder mit einer zumindest im Wesentlichen flachen oder ebenen Oberseite mit dem Detektions- bzw. Bindungsbereich versehen. Dies ist einer guten bzw. definierten Detektion bzw. Bestimmung eines Analyten zuträglich.

Gemäß einem weiteren Aspekt der vorliegenden Erfindung ist der Träger in einer definierten Ausrichtung in dem oder von dem Kanal geführt. Insbesondere ist der Träger in dem Kanal nicht umdrehbar, sondern weist vorzugsweise mit einer Flachseite und/oder seiner den Detektions- bzw. Bindungsbereich enthaltenden Seite zu einer Seite (insbesondere Flachseite) der Vorrichtung bzw. des Kanals. Dies gestattet eine besonders gute, insbesondere optische Detektion von der letztgenannten Seite her und vereinfacht damit den Aufbau der Vorrichtung bzw. die Detektion generell.

Gemäß einem anderen Aspekt der vorliegenden Erfindung weist der Träger eine Länge auf, die größer als der maximale Querschnitt des Kanals ist. Dies gestattet eine wesentlich definiertere Bewegung und/oder Ausrichtung des Trägers in dem Kanal bzw. mikrofluidischen System als beim Stand der Technik. So kann ein wesentlich definierterer Verfahrensablauf und/oder eine bessere bzw. definiertere, insbesondere optische Detektion erfolgen.

Gemäß einem weiteren Aspekt der vorliegenden Erfindung füllt der Träger den Querschnitt des Kanals zu mehr als 30 %, vorzugsweise mehr als 40 %, insbesondere mehr als 50 % flächenmäßig aus. Hierdurch können die erforderlichen Volumina minimiert und/oder die auftretenden Diffusionslängen bzw. Reaktionszeiten reduziert werden.

Gemäß einem weiteren Aspekt der vorliegenden Erfindung ist der Träger formstabil mit einer definierten Form ausgebildet. Dies ist wiederum einer definierten Detektion zuträglich.

Gemäß einem weiteren Aspekt der vorliegenden Erfindung ist nur ein einziger Träger zur Detektion und/oder in dem Kanal vorgesehen. Dies ist einer definierten Detektion zuträglich.

Gemäß einem weiteren Aspekt der vorliegenden Erfindung ist der Träger als ein Kunststoffteil und/oder Spritzgussteil ausgebildet. Dies gestattet eine einfache, kostengünstige Herstellung und/oder die Realisierung definierter Eigenschaften und/oder eine Erleichterung der Massenproduktion.

Gemäß einem weiteren Aspekt der vorliegenden Erfindung ist der Detektions- bzw. Bindungsbereich zumindest teilweise mikrostrukturiert. Unter "Mikrostrukturierung" ist vorzugsweise eine sich insbesondere wiederholende Struktur und/oder eine Struktur mit einer mittleren Strukturbreite von 10 nm bis 500 µm, vorzugsweise weniger als 10 µm, und/oder mit Erhebungen oder Vertiefungen von 10 nm bis 500 µm, vorzugsweise weniger als 10 µm, zu verstehen. Die Mikrostrukturierung dient einer Oberflächenvergrößerung und/oder erleichtert das Binden eines Stoffes, wie eines Analyten oder Reagenzes, insbesondere eines Antikörpers oder dergleichen, im Detektions- bzw. Bindungsbereich.

Gemäß einem weiteren Aspekt der vorliegenden Erfindung kann der Träger in verschiedene Flüssigkeiten, beispielsweise von der flüssigen Probe in eine andere Flüssigkeit oder umgekehrt, und/oder wahlweise in verschiedene Reaktionskammern bewegbar sein. Dies ist wiederum einer optimierten Reaktionsführung, einem optimierten Ablauf verschiedener Schritte und/oder einer optimierten Detektion zuträglich.

Die genannten Aspekte der vorliegenden Erfindung sowie die sich aus der nachfolgenden Beschreibung und den Ansprüchen ergebenden sonstigen Aspekte und Merkmale der vorliegenden Erfindung können unabhängig voneinander, aber auch in beliebiger Kombination realisiert werden.

Weitere Aspekte, Merkmale, Eigenschaften und Vorteile der vorliegenden Erfindung ergeben sich aus den Ansprüchen und der nachfolgenden Beschreibung bevorzugter Ausführungsformen anhand der Zeichnung. Es zeigt:
- Fig. 1: eine schematische Draufsicht einer ersten Ausführungsform einer vorschlagsgemäßen Vorrichtung;
- Fig. 2: einen schematischen Längsschnitt der Vorrichtung;
- Fig. 3: einen schematischen Querschnitt der Vorrichtung;
- Fig. 4: eine schematische Draufsicht einer zweiten Ausführungsform der vorschlagsgemäßen Vorrichtung; und
- Fig. 5: eine schematische Schnittdarstellung einer Testeinrichtung mit der vorschlagsgemäßen Vorrichtung.

In den Figuren werden für gleiche oder ähnliche Bauteile und Komponenten die gleichen Bezugszeichen verwendet, wobei sich entsprechende oder ähnliche Vorteile und Eigenschaften ergeben, auch wenn eine wiederholte Beschreibung weggelassen ist.

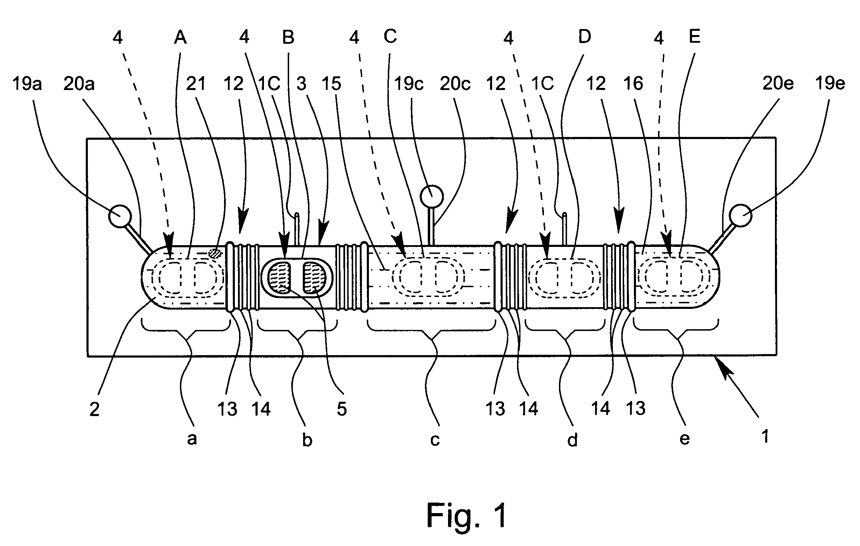

Fig. 1 zeigt in einer schematischen Draufsicht eine vorschlagsgemäße Vorrichtung 1 zur Manipulation oder Untersuchung einer flüssigen Probe 2. Die Vorrichtung 1 ist vorzugsweise zumindest im Wesentlichen kartenartig, plattenartig, flach, dünn und/oder eben ausgebildet.

Die Vorrichtung 1 weist vorzugsweise (mindestens) einen insbesondere mikrofluidischen Kanal 3 zur Aufnahme der Probe 2 auf. Der Kanal 3 oder die Vorrichtung 1 bildet insbesondere ein mikrofluidisches System oder einen Teil davon.

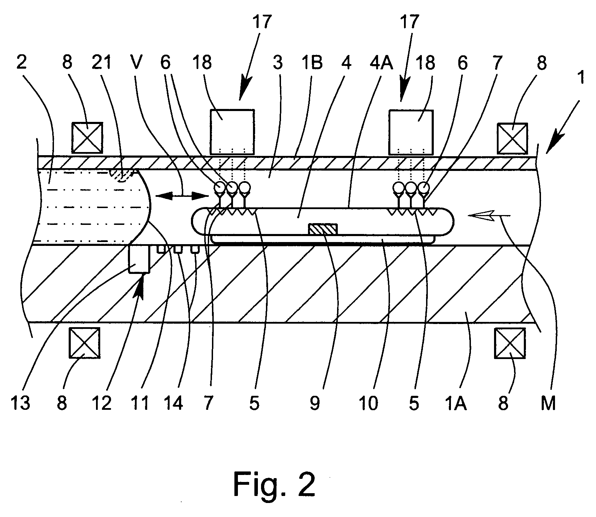

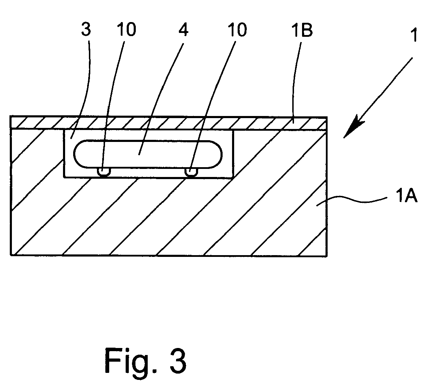

Wie in einem schematischen Längsschnitt der Vorrichtung 1 gemäß Fig. 2 und einem schematischen Querschnitt der Vorrichtung 1 gemäß Fig. 3 angedeutet, weist die Vorrichtung 1 vorzugsweise ein Basisteil 1A auf, in dem der Kanal 3 bzw. das mikrofluidische System insbesondere durch eine oder mehrere Vertiefungen - bevorzugt in Form einer Nut oder mehrerer Nuten - gebildet ist bzw. sind, die durch einen Deckel 1B abgedeckt ist bzw. sind.

Das Basisteil 1A ist vorzugsweise als Kunststoffteil und/oder Spritzgussteil ausgebildet. Das Basisteil 1A ist vorzugsweise zumindest im Wesentlichen flach, eben, plattenartig und/oder steif ausgebildet. Der Kanal 3 bzw. das mikrofluidische System ist vorzugsweise in oder entlang einer Flachseite des Basisteils 1A gebildet und/oder zu einer Flachseite hin offen. Der Kanal 3 bzw. die Vertiefung bzw. Flachseite ist durch den Deckel 1B vorzugsweise zumindest im Wesentlichen vollständig abgedeckt. Jedoch sind andere konstruktive Lösungen auch möglich.

Der Deckel 1B ist vorzugsweise als Folie ausgebildet. Der Deckel 1B ist vorzugsweise aufgeklebt oder auflaminiert und/oder aufgeschweißt. Vorzugsweise ist der Deckel 1B durch eine Klebefolie oder Heißsiegelfolie oder dergleichen gebildet.

Der Deckel 1B ist vorzugsweise zumindest bereichsweise transparent ausgebildet, insbesondere um eine optische Detektion, wie später noch näher beschrieben, zu ermöglichen.

Die Vorrichtung 1 weist mindestens einen in dem Kanal 3 bzw. System und/oder in der Probe 2 beweglichen Träger 4 auf.

Der Träger 4 ist vorzugsweise in die Probe 2 hinein bewegbar und/oder innerhalb der Probe 2 bewegbar, wie durch die gestrichelte Position A in Fig. 1 angedeutet, um insbesondere eine Untersuchung oder Wechselwirkung mit der Probe 2 bzw. Bestandteilen der Probe 2, wie einem Analyten, zu ermöglichen. Besonders bevorzugt ist der Träger 4 aus der Probe 2 heraus - beim Darstellungsbeispiel gemäß Fig. 1 in einen anderen Bereich des Kanals 3 bzw. Systems - bewegbar, wie durch Position B angedeutet.

Der Träger 4 weist vorzugsweise mindestens einen Detektions- bzw. Bindungsbereich 5, beim Darstellungsbeispiel zwei oder mehrere Detektions- bzw. Bindungsbereiche 5 auf. Nachfolgend wird die Funktion des Detektions- bzw. Bindungsbereichs 5 bzw. der Detektions- bzw. Bindungsbereiche 5 näher erläutert, wobei insbesondere auf die Fig. 2 Bezug genommen wird, die die Vorrichtung 1 in einem schematischen Längsschnitt in Position B zeigt.

Jeder Detektions- bzw. Bindungsbereich 5 wechselwirkt vorzugsweise nur mit einem Stoff. Bei dem Stoff handelt es sich vorzugsweise um einen Analyten der Probe 2 oder ein daraus entstandenes oder abhängiges Produkt, wie ein Komplex oder um einen Komplex oder um ein Reagenz, das mit dem Analyten oder davon abhängigen Produkt wechselwirkt oder bindet. Insbesondere dient ein Detektions- bzw. Bindungsbereich 5 dem Binden eines bestimmten Stoffes, wie eines Bestandteils bzw. Analyten der Probe 2 und/oder eines davon gebildeten Komplexes 6 und/oder dem Binden eines Reagenzes, das mit der Probe 2 bzw. einem Bestandteil oder Analyten der Probe 2 oder einem davon gebildeten Komplex 6 wechselwirkt. Besonders bevorzugt handelt es sich bei dem Reagenz bzw. Stoff gemäß dem Darstellungsbeispiel um einen oder mehrere, ggf. auch unterschiedliche Antikörper 7, die mit einem Analyten oder einem Komplex 6 oder sonstigen Produkt des Analyten wechselwirken bzw. binden.

Vorzugsweise ist der im Detektions- bzw. Bindungsbereich 5 angeordnete oder diesen bindende Stoff oder das Reagenz ein Antikörper 7 oder enthält Antikörper 7. Besonders bevorzugt ist der Detektions- oder Bindungsbereich 5 durch immobilisierte Antikörper 7 gebildet oder bedeckt.

Der Begriff "Detektions- bzw. Bindungsbereich" ist bei der vorliegenden Erfindung vorzugsweise sehr allgemein dahingehend zu verstehen, dass er direkt oder indirekt (z.B. nach Vorbehandlung oder Aufbringen der Antikörper 7) mit dem Stoff, insbesondere einem Analyten, wechselwirkt. Die Wechselwirkung kann insbesondere in einer Reaktion, einem Binden, einer Komplexbildung, einer Adhäsion, einem Anlagern, einem katalytischen Einfluss oder einer beliebigen Kombination davon liegen.

Vorzugsweise weist der Träger 4 mehrere, insbesondere räumlich von einander getrennte Detektions- bzw. Bindungsbereiche 5 auf, die insbesondere mit unterschiedlichen Stoffen Wechselwirken bzw. der Bestimmung unterschiedlicher Analyten der Probe 2 dienen, wie beim Darstellungsbeispiel angedeutet.

Die vorschlagsgemäße Vorrichtung 1 und das vorschlagsgemäße Verfahren dienen also vorzugsweise der Untersuchung bzw. Diagnostik der Probe 2, insbesondere der qualitativen und/oder quantitativen Bestimmung mindestens eines Analyten der Probe 2. Insbesondere wird ein Immunoassay realisiert, besonders bevorzugt als Sandwich-Immunoassay. Jedoch können auch andere Reaktionen ablaufen oder sonstige Substanzen, Reaktionsprodukte, Eigenschaften oder dergleichen bestimmt bzw. gemessen oder erfasst werden.

Alternativ oder zusätzlich können die vorschlagsgemäße Vorrichtung 1 und das vorschlagsgemäße Verfahren auch zur Manipulation der Probe 2, insbesondere durch Bewegen des Trägers 4 innerhalb der Probe 2 und/oder durch Herausbewegen des Trägers 4 aus der Probe 2 und/oder Hineinbewegen des Trägers 4 in die Probe 2 verwendet werden. So kann beispielsweise ein Durchmischen der Probe 2 oder eine Übertragung einer bestimmten Menge der Probe 2 oder eines Bestandteils der Probe 2 realisiert werden. Auch ist durch Bewegen des Trägers 4 eine Übertragung in die Probe möglich.

Zum Bewegen des Trägers 4 weist die Vorrichtung 1 vorzugsweise eine Manipuliereinrichtung 8 auf. Die Manipuliereinrichtung 8 kann der Vorrichtung 1 aber auch zugeordnet sein, insbesondere Teil einer Testeinrichtung bilden, wie später noch erläutert.

Das Bewegen des Trägers 4 erfolgt beim Darstellungsbeispiel vorzugsweise magnetisch, insbesondere also durch Variation eines auf den Träger 4 wirkenden Magnetfelds M. Insbesondere wird das Magnetfeld M extern erzeugt. Beim Darstellungsbeispiel weist die Manipuliereinrichtung 8 hierzu mindestens einen externen Magneten und/oder Elektromagneten oder mehrere Elektromagnete auf, wie in Fig. 2 angedeutet.

Weiter ist der Träger 4 zumindest teilweise aus magnetischem oder magnetisierbarem Material hergestellt oder damit versehen, das insbesondere einen Magneten 9 bildet, wie in Fig. 2 angedeutet. Insbesondere handelt es sich hierbei um ein paramagnetisches, superparamagnetisches oder ferromagnetisches Material.

Das magnetische Material oder der Magnet 9 kann beispielsweise in dem Träger 4 aufgenommen, wie in Fig. 2 angedeutet, oder darin eingeklebt oder daran angebracht oder angeklebt oder beispielsweise darin eingegossen sein. Jedoch kann der Träger 4 auch zumindest im Wesentlichen aus magnetischem oder magnetisierbarem Material hergestellt sein. Bedarfsweise können auch mehrere oder unterschiedliche magnetische Materialien oder Magnete 9 am Träger 4 - beispielsweise räumlich verteilt oder beabstandet voneinander - angebracht oder eingesetzt werden, insbesondere um eine bestimmte Steuerbarkeit oder Bewegbarkeit des Trägers 4 zu erreichen.

Durch das magnetische Material bzw. den Magneten 9 kann beispielsweise erreicht werden, dass der Träger 4 entlang eines Gradienten des auf den Träger 4 wirkenden Magnetfelds M, also insbesondere des von der Manipuliereinrichtung 8 erzeugten Magnetfelds M, weiter insbesondere in einen Bereich hoher Magnetfeldstärke bewegbar ist.

Durch entsprechende Änderung des Magnetfelds M bzw. des Gradienten des Magnetfelds M ist der Träger 4 in eine bestimmte Richtung, in einen bestimmten Bereich und/oder auch in entgegengesetzten Richtungen bewegbar. Durch entsprechend schnelle Änderungen, insbesondere Änderung der Richtung des Gradienten des Magnetfelds M, kann eine gegebenenfalls auch sehr schnelle Hin- und Herbewegung bzw. alternierende Bewegung des Trägers 4 bzw. ein Schwingen des Trägers 4 realisiert werden.

Alternativ oder zusätzlich kann der Träger 4 auch auf jede sonstige Art und Weise, beispielsweise durch Einwirkung der Gewichtskraft, beispielsweise bei Schwenken oder Umdrehen, oder durch Einwirkung einer sonstigen Kraft oder Beschleunigung, beispielsweise bei Rotation der Vorrichtung 1, durch elektrische Anziehung oder Abstoßung, durch reib- oder formflüssigen Angriff am Träger 4, beispielsweise mittels eines Fadens oder eines sonstigen Zugmittels oder Druckmittels, durch Ultraschall oder dergleichen erfolgen.

Der Träger 4 ist vorzugsweise in Längsrichtung bzw. entlang des Kanals 3 zumindest in eine Richtung oder in beide Richtungen bewegbar.

Um ein leichtes und/oder definiertes Bewegen des Trägers - insbesondere im Kanal 3 oder entlang des Kanals 3 oder auf einem Boden des Kanals 3 bzw. auf dem Basisteil 1A - zu erreichen oder zu unterstützen, weist der Träger 4 optional wenigstens ein Führungsmittel 10 auf, dass beim Darstellungsbeispiel durch insbesondere mehrere Vorsprünge oder Kufen gebildet ist. Das Führungsmittel 10 kann bzw. die Vorsprünge oder Kufen können beispielsweise das Gleiten des Trägers 4 auf dem Basisteil 1A bzw. dem Boden des Kanals 3 erleichtern, insbesondere also den Gleitwiderstand reduzieren, und/oder bedarfsweise in eine nicht dargestellte Rille oder Längsnut oder dergleichen eingreifen, insbesondere um den Träger 4 beispielsweise in Längsrichtung des Kanals 3 verschiebbar zu führen. Die Führungsmittel 10 kann den Träger 4 bzw. den oder die Bereiche 5 alternativ oder zusätzlich auch beabstandet zum Boden des Kanals 3, zu Seitenwänden des Kanals 3 und/oder zum Deckel 1B führen oder halten und/oder beabstandet zum Boden des Kanals 3, zu Seitenwänden des Kanals 3 und/oder zum Deckel 1B zu halten.

Der Träger 4 ist vorzugsweise in einer definierten Ausrichtung in dem Kanal 3 oder mittels des Kanals 3 geführt. Dies erleichtert die Detektion.

Der Träger 4 ist vorzugsweise zumindest im Wesentlichen eben oder plattenartig ausgebildet und/oder weist vorzugsweise eine zumindest im Wesentlichen ebene Oberseite mit dem Detektions- oder Bindungsbereich 5 bzw. den Detektions- oder Bindungsbereichen 5 auf. Dies erleichtert insbesondere die Detektion.

Vorzugsweise weist der Träger 4 eine Länge auf, die größer als der maximale Querschnitt des Kanals 3 ist. Dementsprechend wird der Träger 4 zumindest in Längsrichtung durch den Kanal 3 geführt.

Vorzugsweise ist der Kanal 3 zumindest im wesentlichen flach oder abgeflacht im Querschnitt ausgebildet, insbesondere so dass der Träger 4 nicht im Kanal 3 umdrehbar ist, insbesondere aufgrund der gegenüber der verhältnismäßig geringen lichten Höhe des Kanals 3 eine demgegenüber größere Breite aufweist.

Besonders bevorzugt füllt der Träger 4 den Querschnitt des Kanals 3 zu mehr als 30% flächenmäßig aus, insbesondere zu mehr als 40 % oder 50 %, wie schematisch in dem Querschnitt gemäß Fig. 3 angedeutet. Hierdurch wird die Detektion erleichtert. Weiter können dadurch die Fusionslängen reduziert und/oder erforderliche Probenvolumina minimiert werden.

Der Träger 4 ist vorzugsweise formstabil mit einer definierten Form ausgebildet. Dies ist wiederum einer definierten Detektion zuträglich.

Besonders bevorzugt ist der Träger 4 oder zumindest dessen Oberfläche mit dem Bereich 5 oder den Bereichen 5 aus einem nicht luminizierenden und bzw. nicht fluoreszierenden Material hergestellt oder damit beschichtet. Dies erleichtert die Detektion von im Bereich 5 verbundenen Stoffen.

Vorzugsweise ist der Träger 4 oder dessen Oberfläche mit dem mindestens einen Bereich 5 zumindest im Wesentlichen dunkel oder besonders bevorzugt schwarz ausgebildet. Dies ist einer optischen Detektion zuträglich.

Der Träger 4 ist vorzugsweise sehr dünn ausgebildet. Insbesondere beträgt seine Dicke (ohne Führungsmittel 10) im Wesentlichen zwischen 10 µm und 500 µm. Die Länge und/oder Erstreckung in Bewegungseinrichtung V des Trägers 4 beträgt vorzugsweise zwischen 1 mm und 10 mm. Die Breite des Trägers 4 beträgt vorzugsweise zwischen 0,5 mm und 5 mm. Vorzugsweise entspricht die Länge des Trägers 4 seiner Haupterstreckung.

Vorzugsweise ist die Breite des Trägers 4 zumindest um einen Faktor 2, insbesondere um einen Faktor 3 oder mehr größer als seine Dicke. Weiter entspricht vorzugsweise die Länge des Trägers 4 zumindest dem 1,5-fachen, insbesondere zumindest dem 2-fachen oder mehr, der Breite des Trägers 4.

Die Längserstreckung des Kanals 3 beträgt vorzugsweise zumindest das doppelte der Länge des Trägers 4, insbesondere zwischen 10 und 100 mm. Der Kanal 3 weist vorzugsweise zumindest im Wesentlichen einen rechteckigen Querschnitt auf oder bildet einen solchen. Die lichte Höhe des Kanals entspricht vorzugsweise zumindest der Dicke des Trägers 4 oder, insbesondere zumindest 10 %, mehr als diese Dicke, insbesondere zwischen 10 µm und 1 mm. Die lichte Weite des Kanals 3 entspricht zumindest der Breite des Trägers 4, insbesondere zumindest 10 %, mehr. Insbesondere kann die lichte Weite des Kanals zwischen 0,5 und 10 mm betragen.

Der Träger 4 ist vorzugsweise zumindest im Wesentlichen in seiner Haupterstreckungsebene - insbesondere in dem Kanal 3 bzw. in dem System - bewegbar. Die verhältnismäßig dünne Ausbildung des Trägers 3 ist dabei einem geringen Strömungswiderstand zuträglich.

Der Träger 4 ist vorzugsweise zumindest im Wesentlichen gradlinig in dem Kanal 3 bewegbar und/oder in dem Kanal 3 hin- und herbewegbar. Es ist jedoch auch möglich, dass der Kanal 3 nicht geradlinig verläuft, sondern beispielsweise gewunden oder mäanderförmig oder in sonstiger Weise verläuft oder abgeknickt ist. Der Träger 4 kann dann vorzugsweise entsprechend des Kanals 3 oder in einem Abschnitt oder Bereich des Kanals 3 bewegt werden.

Vorzugsweise ist in der Vorrichtung 1 bzw. dem Kanal 3 oder dem System nur ein einziger beweglicher Träger 4 vorgesehen. Dies ist einer definierten Bewegung und/oder Detektion zuträglich.

Der Träger 3 ist vorzugsweise aus Kunststoff hergestellt bzw. ein Kunststoffteil. Dies gestattet eine günstige Herstellung bzw. die Verwendung von Material mit gewünschten Eigenschaften. Beispielsweise ist der Träger 4 zumindest im Wesentlichen aus PMA oder PE hergestellt.

Der Träger 3 ist vorzugsweise ein Spritzgussteil bzw. durch Spritzgießen hergestellt. Dies gestattet eine einfache und kostengünstige Herstellung und/oder Formung.

Vorzugsweise ist zumindest die Oberseite oder Flachseite des Trägers 4 mit dem mindestens einen Detektions- oder Bindungsbereich 5 zumindest im Wesentlichen eben oder glatt ausgeführt. Die Oberseite oder der Detektions- bzw. Bindungsbereich 5 kann jedoch auch - je nach Bedarf - mikrostukturiert oder in sonstiger Weise strukturiert sein. Die Strukturierung ist beispielsweise in dem schematischen Schnitt gemäß Fig. 2 in den Bereichen 5 angedeutet. Die Mikrostrukturierung kann beispielsweise das Binden von Reagenzien, wie der Antikörper 7, erleichtern.

Hinsichtlich des Bindens der Antikörper 7 oder sonstiger Reagenzien ist anzumerken, dass besonders bevorzugt eine Plasmabehandlung des Trägers 4 bzw. dessen Oberseite oder Flachseite und/oder der Bereiche 5 und besonders bevorzugt ein Immobilisieren der Antikörper 7 auf den Träger 4 bzw. in den Bereichen 5 erfolgt.

Der Träger 4 ist vorzugsweise in verschiedene Bereiche des Kanals 3 bzw. mikrofluidischen Systems und/oder in verschiedene Flüssigkeiten bewegbar. Dies wird nachfolgend näher erläutert.

Der Träger 4 ist beim Darstellungsbeispiel zunächst vorzugsweise innerhalb der Probe 2 bewegbar, beispielsweise hin- und hergehend bzw. alternierend und/oder daran in Schwingung versetzbar. Der Bereich a des Kanals 3 bzw. des Systems, in dem sich die Probe 2 befindet, bildet vorzugsweise eine Reaktionskammer. Vorzugsweise befindet sich die Probe 2 nur in diesem Kanalbereich a, ist also auf einen Teil des Kanals 3 beschränkt.

Der Kanal 3 bzw. das System weist vorzugsweise einen Bereich b auf, der einen Gasraum bildet, also nicht mit der Probe 2 oder einer sonstigen Flüssigkeiten gefüllt ist. Der Träger 4 ist vorzugsweise von dem Bereich a in den Bereich b, also von Position A nach Position B, bewegbar und/oder umgekehrt, vorzugsweise mittels der Manipuliereinrichtung 8. Insbesondere ist der Träger 4 aus der flüssigen Probe 2 heraus oder in diese hinein oder über eine Phasengrenze flüssig-gas oder gas-flüssig 11 bewegbar, die in Fig. 2 angedeutet ist. Insbesondere ist der Träger 4 aus der Probe 2 bzw. aus der Reaktionskammer heraus in den Gasraum oder umgekehrt bewegbar. Die Detektion erfolgt besonders bevorzugt in dem Gasraum bzw. außerhalb einer Flüssigkeit. Dies ist insbesondere einer optischen Detektion zuträglich.

Um die Probe 2 bzw. Flüssigkeit in dem Bereich a zu halten ist vorzugsweise ein Mittel 12 vorgesehen, um Flüssigkeit zurückzuhalten. Bei dem Mittel 12 handelt es sich insbesondere um einen Kapillarstopp bzw. eine abrupte Querschnittserweiterung, beispielsweise durch einen insbesondere sowohl im Boden als auch in Seitenwänden des Kanals 3 gebildeten Graben 13. Das Mittel 12 kann jedoch zusätzlich der alternativ auch durch einen Oberflächenbereich mit unterschiedlichen Oberflächeneigenschaften aufweisen, der beispielsweise hydrophob ausgebildet sein kann, wenn die zurückzuhaltende Probe 2 oder sonstige Flüssigkeit hydrophil ist, oder umgekehrt.

Alternativ oder zusätzlich dient das Mittel 12 einem "Abstreifen" der Flüssigkeit bzw. Probe 2 von dem Träger 4, wenn dieser aus der Flüssigkeit bzw. Probe 2 heraus - insbesondere direkt in einen Gasraum und/oder über eine Phasengrenze 11 - bewegt wird. Das Mittel 12 kann insbesondere zu diesem Abstreifen nacheinander mehrere Bereiche mit verringerter Kapillarität bzw. unterschiedlichen Oberflächeneigenschaften, beispielsweise mehrere Rillen, Nuten 14 oder dergleichen, aufweisen, die beim Darstellungsbeispiel vorzugsweise kleiner als der Graben 13 ausgebildet sind. Jedoch sind hier auch andere konstruktive Lösungen möglich.

Das Mittel 12 ist vorzugsweise zwischen dem Bereich a und b bzw. im Bereich des Übergangs von dem Bereich a bzw. Probe 2 zu dem Bereich b bzw. dem Gasraum angeordnet. Vorzugsweise ist der Graben 13 mit der deutlich verringerten Kapillarität bzw. der Kapillarstopp direkt unmittelbar benachbart zu der Reaktionskammer bzw. dem Bereich b bzw. zur Probe 2 angeordnet, wobei sich die weiteren Mittel zum Abstreifen von Flüssigkeit bzw. Nuten 14 oder dergleichen dann vorzugsweise auf der der Flüssigkeit bzw. Probe 2 gegenüberliegenden Seite - also zum Gasraum hin - an den Graben 13 oder dergleichen anschließen.

Wie bereits erwähnt, ist der Träger 4 vorzugsweise in verschiedene Positionen bewegbar. Besonders bevorzugt ist der Träger 4 mit verschiedenen Flüssigkeiten in Kontakt bringbar. An Stelle eines Wechselns der Flüssigkeit erfolgt vorschlagsgemäß vorzugsweise ein entsprechendes Bewegen des Trägers 4 bzw. eine Änderung der Position des Trägers 4. Insbesondere ist der Träger 4 von der Probe 2 in eine davon verschiedene Flüssigkeit 15, beispielsweise eine Waschflüssigkeit, oder umgekehrt bewegbar, wie in Fig. 1 angedeutet.

Beim Darstellungsbeispiel ist die Flüssigkeit 15 vorzugsweise in einem Bereich c des Kanals 3 bzw. mikrofluidischen System bzw. der Vorrichtung 1 aufgenommen oder gehalten. Der Bereich c bildet vorzugsweise eine weitere Reaktionskammer.

Der Träger 4 ist vorzugsweise ausgehend von der Position a bzw. aus der Probe 2 insbesondere über den Bereich b bzw. den Gasraum in die weitere Flüssigkeit 15 bzw. Reaktionskammer und/oder den Bereich c bewegbar, wie durch Position C in Fig. 1 angedeutet. Der Träger 4 ist also beispielsweise aus der Position A über die Position B in die Position C bewegbar. Jedoch kann der Träger 4 grundsätzlich auch entgegensetzt bewegbar sein.

Zwischen dem Gasraum und dem Bereich c ist vorzugsweise wiederum ein Mittel 12 zum Zurückhalten der Flüssigkeit 15 vorgesehen, wie in Fig. 1 angedeutet.

Beim Darstellungsbeispiel ist der Bereich b bzw. der Gasraum zwischen dem Bereich a bzw. der davon gebildeten Reaktionskammer und/oder der Probe 2 einerseits und dem Bereich c bzw. der davon gebildeten Reaktionskammer und/oder der Flüssigkeit 15 andererseits angeordnet. Jedoch kann der dazwischen angeordnete Gasraum oder Bereich b auch entfallen oder stark verkürzt sein, beispielsweise nur durch ein Mittel 12 bzw. nur durch einen größeren Graben 13 oder dergleichen gebildet sein. Insbesondere kann der Gasraum bzw. können die Mittel 12 zwischen den Bereichen a und c derart verkürzt sein, dass der Bereich b quasi entfällt und/oder kürzer als die Länge des Trägers 4 wird.

Alternativ ist es auch möglich, dass das Mittel 12 bzw. die Nuten 14 oder dergleichen sich über eine Strecke in Bewegungsrichtung des Trägers 4 bzw. Längserstreckung des Kanals 3 erstrecken, die im wesentlichen der Länge des Trägers 4 entspricht oder insbesondere deutlich länger ist.

Bedarfsweise kann das Mittel 12 zwischen der Probe 2 und Flüssigkeit 15 bzw. zwischen zwei sonstigen Flüssigkeiten auch ganz entfallen, insbesondere wenn die Flüssigkeiten nicht mischbar sind und eine Phasengrenze bilden, beispielsweise durch entsprechende Schichtung, durch die der Träger 4 bewegbar ist.

Bei der ersten Ausführungsform weist die Vorrichtung 1 bzw. der Kanal 3 bzw. das System vorzugsweise bzw. optional über die von der Probe verschiedene Flüssigkeit 15 hinaus eine weitere Flüssigkeit 16 auf, in die der Träger 4 (bedarfsweise) bewegbar ist. Hierzu weist die Vorrichtung 1 bzw. der Kanal 3 bzw. das System vorzugsweise einen weiteren Bereich e zur Aufnahme bzw. zum Halten der Flüssigkeit 16 auf.

Der Träger 4 ist beim Darstellungsbeispiel ausgehend von der Position C bzw. aus dem Bereich c über einen Bereich d, der einen optionalen weiteren Gasraum bildet, bzw. über die Position D in den Bereich e bzw. die Position E, also in die weitere Flüssigkeit 16 bewegbar.

Zwischen den Bereichen c und d einerseits und den Bereichen d und e andererseits sind vorzugsweise wiederum jeweils Mittel 12 bzw. Gräben 13 und/oder Nuten 14 oder dergleichen angeordnet.

Es ist anzumerken, dass die Mittel 12 der Vorrichtung 1 bzw. des Kanals 3 grundsätzlich zumindest im Wesentlichen gleich oder je nach Bedarf, beispielsweise aufgrund Verwendung unterschiedlicher Flüssigkeiten oder dergleichen, auch unterschiedlich ausgebildet sein können.

Beim Darstellungsbeispiel ist der Träger A vorzugsweise ausgehend von der Position A über die Position B, C und D in die Position E bewegbar. Je nach ablaufender Reaktion oder zu sonstigen Zwecken kann die Aufenthaltszeit des Trägers 4 in den einzelnen Position bzw. Bereichen und/oder in den einzelnen Flüssigkeiten bzw. in der Probe 2 beliebig variiert werden. Auch kann ein beliebiges zusätzliches Bewegen des Trägers 4 innerhalb des jeweiligen Bereichs oder um die jeweilige Position herum, also beispielsweise in der Probe 2 und/oder in den Flüssigkeiten 15 bzw. 16 erfolgen.

Wie bereits angedeutet, erfolgt die Detektion besonders bevorzugt in einem Gasraum. Beim Darstellungsbeispiel kann beispielsweise eine Detektion im Bereich b oder d bzw. in der Position B oder D erfolgen. Ggf. kann auch in beiden Bereichen bzw. Positionen jeweils eine Detektion erfolgen, also in mehreren unterschiedlichen Bereichen oder Positionen eine Detektion erfolgen. Bedarfweise können in den unterschiedlichen Bereichen bzw. Position auch unterschiedliche Detektionen erfolgen. So können beispielsweise mehrstufige oder verschiedene Reaktionen nacheinander durchgeführt, detektiert oder überwacht werden.

Wie bereits erwähnt, wird beim Darstellungsbeispiel der Träger 4 vorzugsweise in verschiedene Reaktionskammern bewegt. Es kann auch in einer Reaktionskammer bzw. in einem Bereich die jeweilige Flüssigkeit bei Bedarf gewechselt werden. Beispielsweise kann statt der eine erste Flüssigkeit bildenden Probe 2 vorher oder nachher eine andere Flüssigkeit in den Bereich a gefüllt werden. Zum oder beim Wechseln der Flüssigkeit kann der Träger 4 dann wahlweise entweder in dem Bereich a bleiben oder in einen anderen Bereich, insbesondere in den Gasraum bzw. angrenzenden Bereich b bewegt werden.

Die einzelnen Bereiche können eine unterschiedliche Länge aufweisen. Vorzugsweise sind einzelne oder alle mit Flüssigkeit gefüllten Bereiche, hier die Bereiche a, c und e, jeweils an die Größe bzw. Länge des Trägers 4 derart angepasst, dass der Träger 4 - sofern erwünscht - in ausreichendem Maße innerhalb des jeweiligen Bereichs bzw. innerhalb der jeweiligen Flüssigkeit bewegbar ist, aber andererseits das erforderliche Flüssigkeitsvolumen gering oder sogar minimiert ist. Dies kann durch entsprechende Anpassung der Länge des jeweiligen Bereichs erfolgen. Besonders bevorzugt beträgt die Länge des jeweiligen Bereichs und/oder die Längserstreckung der Mittel 12 vorzugsweise zumindest im wesentlichen jeweils zwischen dem 1,2-fachen und 10-fachen, besonders bevorzugt zwischen dem 1,5-fachen und 3-fachen der Länge des Trägers 4 bzw. Erstreckung des Trägers 4 in Bewegungsrichtung. Aufgrund der Volumenverdrängung durch den Träger 4 kann es möglich oder zielführend sein, das erforderliche Flüssigkeitsvolumen so zu bemessen, dass insbesondere die bevorzugten Längserstreckungen oder eine zumindest im Wesentlichen vollständige Füllung die jeweiligen Bereiche (insbesondere erste) im Zustand des sich in der Flüssigkeit befindlichen Trägers 4 erreicht werden. Auch dies kann zu einer Minimierung des jeweils notwendigen Flüssigkeitsvolumens führen.

Alternativ oder zusätzlich kann eine Minimierung der erforderlichen Flüssigkeitsvolumina dadurch erreicht werden, dass der Träger 4 den Kanal 3 im Querschnitt flächenmäßig zu zumindest 30%, vorzugsweise zu mehr als 40 %, insbesondere zu mehr als 50 %, ausfüllt. Mit anderen Worten, wenn die Breite und/oder Höhe des Kanals 3 an die entsprechenden Dimensionen des Trägers 4 angepasst werden, kann ebenfalls eine Verringerung der erforderlichen Flüssigkeitsvolumina und/oder eine bereits angesprochene Führung des Trägers 4 im Kanal 3 erreicht werden. Besonders bevorzugt ist der Träger im Querschnitt zumindest im Wesentlichen konkludent zu dem Querschnitt des Kanals 3 oder umgekehrt ausgebildet.

Der Träger 4 ist vorzugsweise in Längserstreckung des Kanals 3 bzw. entlang des Kanals 3 bewegbar, wie durch Fall V in Fig. 2 schematisch angedeutet. Alternativ oder zusätzlich kann der Träger 4 jedoch auch quer dazu, insbeson-dere in seiner Flächenerstreckung und/oder parallel zur Flächenerstreckung der Vorrichtung 1 bzw. des Basisteils 1b oder Deckels 1a, vorzugsweise mittels der Manipuliereinrichtung 8 und/oder durch sonstige Einwirkungen, bewegbar sein.

Um die gewünschte Bewegbarkeit des Trägers 4 zu ermöglichen, weist die Manipuliereinrichtung 8 vorzugsweise mehrere entsprechend positionierte Elektromagnete auf, wobei die Elektromagnete insbesondere in erforderlicher Weise steuerbar sind, um das gewünschte Magnetfeld M bzw. den gewünschten Gradienten zu erreichen.

Vorzugsweise ist die Position des Trägers 4 in der Vorrichtung 1 bzw. in dem Kanal 3 oder mikrofluidischen System erfassbar. Dies erfolgt insbesondere induktiv. Beispielsweise kann dies induktiv mittels einer entsprechenden Spule oder mehreren Spulen und/oder mittels der Elektromagnete bzw. der Spulen der Elektromagnete der Manipuliereinrichtung 8 erfolgen. Jedoch kann die Erfassung der Position des Trägers 4 zusätzlich oder alternativ auch auf sonstige Weise, beispielsweise optisch, erfolgen.

Der Kanal 3 weist vorzugsweise einen zumindest im Wesentlichen konstanten Querschnitt und/oder einen zumindest im Wesentlichen glatten oder ebenen Boden oder sonstige Führungsfläche für den Träger 4 auf. Die Mittel 12, Gräben 13 und/oder Nuten 14 oder dergleichen stellen vorzugsweise in Bewegungsrichtung V des Trägers 4 nur relativ kurze Unterbrechungen und/oder Querschnittserweiterungen dar, weisen also eine insbesondere nur kurze Erstreckung in dieser Richtung auf, insbesondere um eine gute Führung des Trägers 4 und/oder ein leichtes Bewegen bzw. Gleiten des Trägers 4 zu ermöglichen oder sicher zu stellen.

Jedoch sind auch andere konstruktive Lösungen möglich. Beispielsweise können vom Basisteil 1A oder im Kanal 3 auch Führungsmittel gebildet sein, die beispielsweise stegartig, schienenartig oder kufenartig ausgebildet sind und/oder vorspringen, um den Gleitwiderstand des Trägers 4 zu verringern und/oder den Träger 4 beispielsweise in Längsrichtung verschiebbar zu führen.

Die Vorrichtung 1 weist vorzugsweise mindestens eine Detektionseinrichtung 17 auf, die insbesondere eine oder mehrere Mess- bzw. Sensoreinrichtungen 18 umfasst, wie in Fig. 2 angedeutet. Die Detektionseinrichtung 17 kann der Vorrichtung 1 aber auch nur zugeordnet sein, insbesondere Teil einer Testeinrichtung bilden, wie später noch erläutert.

Die Detektionseinrichtung 17 ist insbesondere zur Detektion eines Analyten oder Stoffs, wie eines Komplexes 6 des Analyten, gegebenenfalls auch von verschiedenen Stoffen bzw. Komplexen 6 verschiedener Analyten, die an einem Bereich 5 oder in verschiedenen Stoffen bzw. Bereichen 5 des Trägers 4 vorhanden, insbesondere gebunden sind, ausgebildet.

Die Detektionseinrichtung 17 arbeitet vorzugsweise optisch, insbesondere durch eine Lumineszenz- oder Fluoreszenzmessung. Dies ist beispielsweise dadurch möglich, dass die Stoffe oder Komplexe 6 oder die nachzuweisenden Analyte entsprechende optische Eigenschaften oder Marker enthalten. Beispielsweise können mittels der Detektionseinrichtung 17 bzw. mittels entsprechender Mess- bzw. Sensoreinrichtungen 18 auch mehrere verschiedene bzw. unterschiedliche Stoffe bzw. Analyte, insbesondere in oder auf verschiedenen Bereichen 5 bestimmt werden, wie in Fig. 2 nur schematisch angedeutet.

Die Detektion erfolgt vorzugsweise also optisch, insbesondere durch Fluoreszenz- oder Lumineszenzmessung. Jedoch kann zusätzlich oder alternativ auch jede sonstige Detektion erfolgen.

Die Messwerte der Detektionseinrichtung 17 können in der Detektionseinrichtung 17 und/oder separat davon - in einer separaten, nicht dargestellten Auswerteeinheit - ausgewertet und/oder weiterverarbeitet und insbesondere auch angezeigt oder auf sonstige Art und Weise ausgegeben werden. Beispielsweise kann der Gehalt eines bestimmten Analyten oder die Gehalte verschiedener Analyte in der Probe 2 gespeichert, angezeigt und/oder ausgegeben werden.

Wie bereits erwähnt, kann eine qualitative und/oder quantitative Detektion bzw. Bestimmung erfolgen.

Die vorschlagsgemäße Vorrichtung 1 ist insbesondere zur Adaptation und Durchführung miniaturisierter Immunoassays geeignet bzw. vorgesehen.

Bei der vorschlagsgemäßen Vorrichtung 1 handelt es sich insbesondere um ein Cartridge-Konzept.

Die vorschlagsgemäße Vorrichtung 1 ist insbesondere vorgesehen oder geeignet, um Parameter bzw. Analyten in Blut-Plasma, Blut-Serum oder dergleichen als Probe 2 zu messen.

Die Vorrichtung 1 weist vorzugsweise eine Aufnahme 19a für die Probe 2 bzw. Blut oder eine sonstige Körperflüssigkeit oder dergleichen auf. Die Aufnahme 19a ist beispielsweise als Aufnahmeöffnung im Deckel 1b gebildet, kann jedoch auch auf sonstige Weise realisiert sein. Die Aufnahme 19a ist beispielsweise über einen Verbindungskanal 2 mit dem Kanal 3 bzw. dessen Bereich a bzw. der davon gebildeten Reaktionskammer verbunden, um die Probe 2 oder dergleichen insbesondere selbsttätig durch Kapillarkräfte in die Reaktionskammer bzw. in den Bereich a zu leiten und diesen mit der Probe 2 zu füllen. Die Aufnahme 19a bzw. Vorrichtung 1 kann zusätzlich eine Einrichtung zur Bluttrennung, wie einen Filter, eine Membran oder dergleichen, enthalten, insbesondere um von aufgegebenem Blut oder beispielsweise in die Aufnahme 19a gegebenen Blut oder auf sonstige Weise zugeleitetem Blut vorzugsweise das Blut-Plasma oder Blut-Serum abzutrennen und als Probe 2 in den Bereich a bzw. die davon gebildete Reaktionskammer zu leiten.

Die Vorrichtung 1 gestattet es, den Proben- und Reagenzienbedarf minimal zu halten.

Die vorschlagsgemäße Einrichtung 1 ermöglicht einen einfachen Aufbau, ein einfaches Design, einen einfachen Zusammenbau und/oder ein einfaches Handling.

Die Vorrichtung 1 gestattet eine miniaturisierte Detektion, insbesondere fluorometrische Detektion.

Die vorschlagsgemäße Vorrichtung 1 gestattet die gleichzeitige Erfassung bzw. Bestimmung mehrerer Parameter oder Analyten.

Die vorschlagsgemäße Vorrichtung 1 bildet ein insbesondere in sich geschlossenes System. Vorzugsweise sollen oder müssen außer der Probe 2 bzw. dem Blut oder sonstigen Flüssigkeiten keine weiteren Reagenzien, Stoffe oder dergleichen zugegeben werden.

Besonders bevorzugt gestattet die Vorrichtung 1 die Durchführung klassischer, modifizierter und/oder miniaturisierter Immunoassays, besonders bevorzugt in Form von Sandwich-Assays oder kompetitiver Immunoassays.

Eine Grundidee der vorschlagsgemäßen Vorrichtung 1 bzw. des vorschlagsgemäßen Verfahren liegt darin, einen Festkörper, nämlich den Träger 4, durch einen Hohlraum, insbesondere in Form des Kanals 3, einer sonstigen Kammer oder in einem sonstigen, insbesondere mikrofluidischen System zu bewegen. Das Bewegen erfolgt insbesondere durch einen magnetischen Feldgradienten, wie bereits erläutert.

Nachfolgend wird ein besonders bevorzugter Verfahrensablauf näher erläutert:
Ein definiertes Volumen der Probe 2, vorzugsweise Blut-Plasma, wird über die Aufnahme 19a auf die Vorrichtung 1 aufgegeben. Über den Verbindungskanal 20a wird die Probe 2, z.B. druckgetrieben und/oder durch Kapillarkräfte, in die Reaktionskammer bzw. in den Kanal 3 bzw. den Bereich a transportiert oder geleitet.

Die Reaktionskammer bzw. der Bereich a wird vorzugsweise mit einem definierten Volumen der Probe 2 befüllt.

Die Reaktionskammer bzw. der Bereich a kann bedarfsweise gleichzeitig als Kammer für die Reaktion und Detektion dienen.

In dem Kanal 3 bzw. Bereich a bzw. der Reaktionskammer wird die Probe 2 vorzugsweise mit einem Reagenz 21 in Kontakt gebracht, das besonders bevorzugt in trockener Form vorgehalten wird bzw. sich bereits im Kanal 3 bzw. Bereich a bzw. der Reaktionskammer befindet. Beispielsweise ist das Reagenz im Deckel 1B angeordnet bzw. darauf aufgebracht und eingetrocknet.

Das Reagenz 21 wird durch die Probe 2 gelöst und kann mit der Probe 2 bzw. einem Analyten der Probe 2 reagieren. Das Lösen des Reagenzes 21 kann bei Bedarf durch ein entsprechendes Bewegen des Trägers 4 im Bereich a bzw. in der Reaktionskammer unterstützt werden.

Je nach Bedarf kann sich der Träger 4 beim Einbringen der Probe 2 in die Reaktionskammer bzw. dem Bereich a bereits darin befinden oder erst nachträglich nach dem Einbringen der Probe 2 darin hinein bewegt werden.

Vorzugsweise enthält das Reagenz 21 ein Konjungat für einen zu bestimmenden Analyten der Probe 2. Das Reagenz 21 kann bedarfsweise zumindest im Wesentlichen nur aus dem Konjungat bestimmen und/oder sonstige Stoffe und Substanzen enthalten. Vorzugsweise hat das Reagenz 21 oder das Konjugat zumindest teilweise eine insbesondere fluoreszierende und/oder lumineszierende und/oder sonstige Marker-Eigenschaft.

Das zunächst in trockener Form vorgehaltene Reagenz 21 bzw. Konjungat wird mit der Probe 2 rekonstruiert. Es kann dann mit dem Analyten in der Probe 2 reagieren und insbesondere einen Analyt-Konjungat-Komplex 6 bilden.

Auf der vorzugsweise sichtbaren und/oder insbesondere durch die Detektionseinrichtung analysierbaren Oberfläche 4A bzw. in dem vorzugsweise sichtbaren Detektions- bzw. Bindungsbereich 5 oder in den vorzugsweise sichtbaren Detektions- bzw. Bindungsbereichen 5 sind beim Darstellungsbeispiel besonders bevorzugt Fängerantikörper 7 oder sonstige mit dem Komplex 6 reagierende Substanzen vorzugsweise immobilisiert. Durch die Mobilität bzw. Beweglichkeit des Trägers 4, durch die geometrische Freiheit bei der Gestaltung und Anordnung der Bereiche 5 und/oder durch die gesteuerte Bewegung, insbesondere mittels eines Magnetfelds M bzw. magnetischen Feldgradienten ergeben sich viele Handlings- und Funktionsmöglichkeiten.

Durch die einheitlichen, definierten und/oder reproduzierbaren Bewegungsmöglichkeiten des Trägers 4 kann die Probe 2 und das Konjungat beispielsweise ideal homogenisiert und zur Reaktion gebracht werden. Weiter kann eine optimale Komplexbildung oder sonstige Reaktion erfolgen. Hierbei kann der Träger 4 gleichzeitig die Funktion eines Mischers einnehmen.

Weiter kann in einem zweiten Reaktionsschritt ein Sandwich-Komplex gebildet werden. Der Analyt-Konjungat-Komplex 6 reagiert dabei mit den immobilisierten Antikörpern 7 auf dem Träger 4 und bildet einen Sandwich-Komplex (Fängerantikörper - Analyt, Detektor-Antikörper). Dieser Zustand ist schematisch in Fig. 2 dargestellt, wobei hier der Träger 4 bereits aus der Probe 2 bzw. aus dem Bereich A heraus in einen anderen Bereich, insbesondere in den Bereich b bzw. einen Gasraum bewegt worden ist.

Die Konjungate können als auch Detektorantikörper bezeichnet werden bzw. solche enthalten oder daraus gebildet sein, insbesondere da sie mit dem zu bestimmten Analyten der Probe 2 spezifisch reagieren bzw. binden sollen.

Das Reagenz 21 bzw. Konjungate enthalten vorzugsweise einen optischen Marker bzw. eine optisch aktive Substanz die ggf. auch durch den Detektorantikörper selbst gebildet sein kann.

Nach der Sandwich-Reaktion bzw. Binden der Komplexe 6 an dem Träger 4 oder einer sonstigen Nachweisreaktion liegt meistens oder immer ein Überschuss an Reagenz 21 bzw. an Konjungaten, Markern, optisch aktiven Substanzen oder dergleichen in der Probe 2 und/oder auf dem Träger 4 vor. Dieser nicht (richtig) gebundene Überschuss soll für eine spätere Detektion, insbesondere zur genauen quantitativen Bestimmung des Analyten, entfernt werden. Dies kann vorzugsweise durch Waschen bzw. eine Waschflüssigkeit, insbesondere einen Waschpuffer, erfolgen.

Gemäß einer Ausführungsvariante wird die Waschflüssigkeit in die Reaktionskammer bzw. den Bereich a eingeleitet und dadurch die Probe 2 verdrängt bzw. entfernt, beispielsweise über einen nicht dargestellten Überlaufkanal in eine nicht dargestellte Überlaufkammer oder dergleichen. Bei diesem Vorgang wird gleichzeitig der Träger bzw. dessen Oberfläche 4A bzw. der Bereich 5 gewaschen. Durch ein Bewegen des Trägers 4 in der Waschflüssigkeit, insbesondere durch hin- und herbewegen, kann ein besonders effektives Waschen erreicht werden.

Das Einleiten der Waschflüssigkeit kann beispielsweise durch Druckluft und/oder Kapillarkraft und/oder auf sonstige Weise erfolgen.

Die Probe 2 kann bedarfsweise auch vorher, also vor Einleiten der Waschflüssigkeit, entfernt bzw. abgeleitet werden.

Gemäß einer anderen Ausführungsvariante wird die Waschflüssigkeit in einer separaten Reaktionskammer bzw. in einem separaten Bereich c der Vorrichtung 1 vorgehalten bzw. aufgenommen. In diesem Fall bildet beispielsweise die Flüssigkeit 15 und/oder 16 eine solche Waschflüssigkeit oder eine sonstige, von der Probe 2 verschiedene bzw. sich unterscheidende Flüssigkeit. Der Träger 4 wird dann dementsprechend durch Bewegen in die Flüssigkeit 15 und/oder 16 bzw. in die entsprechenden Bereiche c und/oder e gewaschen bzw. sonstigen Reaktionen oder Behandlungen unterzogen.

Das Einfüllen der Flüssigkeiten 15 bzw. 16 erfolgt vorzugsweise über entsprechende Aufnahmen 19c bzw. 19e und Verbindungskanäle 20c und 20e. Das Befüllen kann insbesondere in entsprechender Weise wie das Befüllen der Vorrichtung 1 mit der Probe 2 erfolgen, so dass die diesbezüglichen Ausführungen entsprechend gelten. Die Aufnahmen 20c und 20e sind vorzugsweise wiederum durch Einfüllöffnungen im Deckel 1b gebildet. Das Überführen der Flüssigkeiten 15 bzw. 16 in die entsprechenden Bereiche c bzw. e bzw. davon gebildeten Reaktionskammern erfolgt wiederum vorzugsweise durch Kapillarkräfte, ggf. durch Druckluft und/oder auf sonstige geeignete Art und Weise.

Bei den Flüssigkeiten 15 und 16 kann es sich grundsätzlich um unterschiedliche Flüssigkeiten handeln. Jedoch kann es sich auch um die gleiche Flüssigkeit, beispielsweise eine Waschflüssigkeit, handeln. In diesem Fall können die beiden Bereiche c und e bzw. die davon gebildeten Reaktionskammern bedarfsweise auch über eine gemeinsame Aufnahme mit der Flüssigkeit befüllbar sein.

Nach den entsprechenden Reaktionen und dem vorzugsweise vorgesehenen Waschen und/oder sonstigen Behandlungen erfolgt die Detektion. Die Detektion erfolgt insbesondere optisch, besonders bevorzugt von der Seite her, zu der die Oberfläche 4A bzw. der Bereich 5 weist, auf dem detektiert wird. In diesem Zusammenhang ist anzumerken, dass bei den vorzugsweise vorgesehenen mehreren Detektions- oder Bindungsbereichen 5 diese besonders bevorzugt jeweils auf einer gemeinsamen Flachseite, Oberfläche 4A oder Oberseite des Trägers 4 angeordnet sind. Jedoch ist auch eine grundsätzlich andere Anordnung möglich. Beispielsweise können auch auf entgegengesetzten Seiten des Trägers 4 separate bzw. verschiedene Bereiche 5 gebildet sein. Ggf. kann auch eine Detektion von verschiedenen Seiten und/oder an verschiedenen Stellen erfolgen. Des Weiteren können die verschiedenen Bereiche 5 simultan oder nacheinander für Reaktionen bzw. zum Binden von Analyten oder dergleichen und/oder zur Detektion verwendet werden.

Bei der vorschlagsgemäßen Vorrichtung 1 ist grundsätzlich eine Detektion wahlweise durch das Basisteil 1A oder durch den Deckel 1B hindurch möglich. Beim Darstellungsbeispiel erfolgt die Detektion vorzugsweise durch den Deckel 1B hindurch. Der Deckel 1B ist dementsprechend zumindest im erforderlichen Detektionsbereich ausreichend transparent ausgebildet. Der Träger 4 weist mit seinem Bereich 5 bzw. seinen Bereichen 5 vorzugsweise dementsprechend zu dem Deckel 1B hin.

Um bei der Detektion möglichst wenig Phasenübergange (gasförmig-festflüssig-fest) zu realisieren, kann der Träger 4 bzw. dessen Detektions- oder Bindungsbereich 5 oder können mehrere oder alle Detektions- oder Bindungsbereiche 5 gegen die vorzugsweise transparente Wandung gedrückt werden. Insbesondere ist ein Drücken gegen den Deckel 1A oder umgekehrt möglich. Der Deckel 1A kann insbesondere hier zu beispielsweise elastisch verformbar, insbesondere folienartig ausgeführt sein. Dies gestattet insbesondere den Einfluss von Flüssigkeit auf die optische Detektion zu verringern oder minimieren, wenn sich der Träger 4 für die Detektion noch in der Flüssigkeit befindet bzw. noch mit (Resten von) Flüssigkeit bedeckt ist. Auch können Störungen durch Übergänge von und/oder zur Gasphase vermieden werden. Gleichzeitig ergibt sich hierdurch eine besonders präzise Positionierung und/oder Fokussierung der Oberfläche für das optische Messen, insbesondere die bevorzugte Lumineszenz- oder Fluoreszenzmessung.

Gemäß einer besonders bevorzugten Variante erfolgt die Detektion bzw. das optische Messen in einem Gasraum, also wenn der Träger 4 zuvor aus der jeweiligen Flüssigkeit, wie der Probe 2, Flüssigkeit 14 oder 16, herausbewegt worden ist. Auch hierdurch können unerwünschte Flüssigkeitseinflüsse minimiert werden. Des Weiteren ist auch in diesem Fall das vorgenannte relative Andrücken des Trägers 4 mit seinem oder seinen Bereichen 5 an die für die Messung vorgesehene, insbesondere transparente Wandung bzw. den Deckel 1A möglich.

Die Detektion erfolgt vorzugsweise optisch bzw. durch Messung oder Bestimmung der Lumineszenz, vorzugsweise der Fotolumineszenz, insbesondere der Fluoreszenz. Die Detektionseinrichtung 17 bzw. deren Mess- oder Sensoreinrichtung 18 ist dementsprechend ausgebildet und umfasst beispielsweise einen CCD-Sensor oder sonstigen Fotosensor oder dergleichen. Weiter fasst die Detektionseinrichtung 11 vorzugsweise auch eine entsprechende Lichtquelle oder dergleichen.

Beim Darstellungsbeispiel kann die bevorzugt in einem Gasraum erfolgende Detektion beispielsweise im Bereich b und/oder d erfolgen. Alternativ oder zusätzlich kann auch eine Detektion in Flüssigkeit, also insbesondere im Bereich a, c und/oder e erfolgen.

Zu Kalibrationszwecken und/oder Erfassung der Position des Trägers 4 kann der Träger 4 mit entsprechenden Markierungen, optisch detektierbaren Bereichen, insbesondere fluoreszierenden Bereichen oder dergleichen versehen sein.

Die Detektions- bzw. Messwerte können in der Detektionseinrichtung 11 selbst und/oder in einer nachgeordneten Auswertung ausgewertet und insbesondere in Form eines Gehalts des zu detektierenden Analyten in der Probe 2 ausgegeben werden. Bei unterschiedlichen zu detektierenden Analyten können entsprechend verschiedene Gehalte oder sonstige verschiedene Parameter ausgegeben werden.

Das Andrücken des Trägers 4 an die transparente Wandung bzw. den Deckel 1B kann auch durch die Manipuliereinrichtung 8 bzw. magnetisch erfolgen.

Die vorschlagsgemäße Vorrichtung 1 und das vorschlagsgemäße Verfahren führen zu verschiedenen Vorteilen:
Der Träger 4 ist vorzugsweise kontaktfrei, insbesondere magnetisch bewegbar.
Der Träger 4 ist in alle Raumrichtungen und/oder rotatorisch bewegbar.
Der Träger 4 ist definiert bewegbar.
Der Träger 4 gestattet sehr große Detektions- oder Bindungsbereiche 5.
Der vorschlagsgemäße Träger 4 gestattet eine besondere Empfindlichkeit und Sensitivität bei der Detektion.
Es werden optimierte Reaktions- und Prozessbedingungen, insbesondere für einen Immunoassay ermöglicht.
Es ist kein präzises Handling von flüssigen Reagenzien erforderlich.
Es können mehrere Parameter bzw. Analyte gleichzeitig, insbesondere durch partiell unterschiedliche Immobilisierungen bzw. Antikörper 7, Bereich 5, und/oder durch unterschiedliche Träger 4, untersucht, insbesondere bestimmt oder detektiert, werden.
Es wird ein einfacher und/oder kostengünstiger Aufbau ermöglicht.
Es werden ein einfacher Zusammenbau und eine einfache Einbringung von Reagenzien, Flüssigkeiten oder dergleichen ermöglicht.
Der Träger 4 ist sehr einfach in verschiedene Flüssigkeiten und/oder Reaktionskammern bewegbar bzw. überführbar.
Verschiedene Flüssigkeiten können durch Gasphasen bzw. Gasräume voneinander getrennt werden.
Der Träger 4 kann für verschiedene Funktionen eingesetzt werden, insbesondere als Transporter und/oder Träger von Molekülen und sonstigen Stoffen, insbesondere biofunktionalen und/oder spezifischen Molekülen.
Auf dem Träger 4 können unterschiedliche Bindungsreaktionen stattfinden, insbesondere zum Entfernen interferierender Stoffe, zum Binden von zu messenden Molekülen, Analyten oder dergleichen. Insbesondere kann es zu beliebigen Interaktionen oder Wechselwirkungen zwischen beispielsweise einer Beschichtung des Trägers 4 und Bestandteilen der zu untersuchenden Probe 2 kommen, die detektierbar sind.
Der Träger 4 kann insbesondere durch eine hin- und hergehende bzw. alternierende Bewegung und/oder eine rotatorische Bewegung als Mischer bzw. Mischelement von Flüssigkeiten, zur Phasenhomogenisierung oder dergleichen eingesetzt werden.
Der Träger 4 bildet eine bewegliche Oberfläche zur Durchführung von insbesondere mehrstufigen Reaktionen und/oder zur Detektion.
Der Träger 4 kann als Ventilelement mit Barrierefunktion zum kontrollierten Öffnen und Schließen von Abschnitten und Bereichen eingesetzt werden, insbesondere um Flüssigkeit kontrolliert freizusetzen und/oder deren Strömung zu steuern.

Das vorschlagsgemäße Verfahren und die vorschlagsgemäße Vorrichtung 1 sind universell einsetzbar und gestatten insbesondere den kontrollierten Ablauf von mehrstufigen Reaktionen und/oder die Detektion von verschiedensten Stoffen oder Analyten und/oder eine Bestimmung verschiedenster Parameter.

Die Vorrichtung 1 bzw. das vorschlagsgemäße Verfahren kann auch Teil eines größeren mikrofluidischen Systems oder Ablauf bilden.

Beispielsweise ist auch möglich, dass die Vorrichtung 1, der Kanal 3 oder das insbesondere mikrofluidische System mehrere, insbesondere zwei oder mehr Träger 4 aufweist, die wahlweise beispielsweise gleichzeitig oder nacheinander in die Probe 2 bzw. die Reaktionskammer oder den Bereich 2 und/oder in einen Gasraum oder eine sonstige Reaktionskammer bzw. Flüssigkeit 15 oder 16 bewegbar sind. Die Träger 4 dienen dann insbesondere der Detektion bzw. Bestimmung unterschiedlicher Analyten und/oder Parameter oder dergleichen.

Die Vorrichtung 1 bzw. der Kanal 3 ist vorzugsweise mit einer geeigneten Entlüftung versehen. Insbesondere ist hier zumindestens ein Lüftungskanal 1C vorgesehen, der insbesondere an einem Gasraum angeschlossen ist. Beim Darstellungsbeispiel ist jeweils ein separater Entlüftungskanal 1C an einen Gasraum, hier an die Bereiche b und d, angeschlossen. Je nach Reihenfolge der Befüllung der Vorrichtung 1 bzw. des Kanals 3 mit der Probe und/oder den Flüssigkeiten 15 und 16 kann auch nur ein einziger Lüftungskanal 1C genügen.

Der Entlüftungskanal 1C kann jeweils wahlweise im Basisteil 1A und/oder Deckel 1B gebildet sein bzw. enden.

Zur Verbesserung der Detektion und/oder Erfassung der Position des Trägers 4 kann insbesondere das Basisteil 1A bzw. der Boden des Kanals 3 dafür günstige Eigenschaften, wie eine bestimmte Farbe oder sonstige Oberflächeneigenschaften, aufweisen.

Gemäß einer nicht dargestellten Ausführungsvariante kann der Träger 4 auch Vertiefungen oder sonstige Mittel aufweisen, um beispielsweise die Probe 2, insbesondere ein bestimmtes Volumen der Probe 2, transportieren und/oder aufnehmen zu können.

Weiter kann der Träger 4 gemäß einer nicht dargestellten Ausführungsvariante auch selbst als mikrofluidisches System mit mindestens einem Kanal, einer Kammer und/oder einem Reservoir für ein Reagenz oder dergleichen, ausgebildet sein.

Gemäß einer weiteren Ausführungsvariante kann der Träger 4 auch dem Transport mindestens eines Reagenzes, beispielsweise des Reagenzes 21, dienen. Beispielsweise kann der Träger 4 mit den eingetrockneten Reagenz 21 versehen sein, das sich beim Einbringen des Trägers 4 in die Probe 2 oder eine sonstige Flüssigkeit in gewünschter Weise löst und dann beispielsweise reagieren kann.

Besonders bevorzugt ist der Träger 4 mit allen Reagenzien 21, Antikörpern 7 und/oder sonstigen Stoffen oder Substanzen versehen oder ausgerüstet, die zur Bestimmung oder Detektion von einem bestimmten Analyten oder mehrerer bestimmter Analyte oder Parameter oder dergleichen erforderlich sind. So kann je nach Bedarf bzw. gewünschter Detektion ein passender Träger 4 in die Vorrichtung 1 bzw. den Kanal 3 eingesetzt werden. Auch können so verschiedene Träger 4 in einem Kanal 3 bzw. einem mikrofluidischen System zur Bestimmung unterschiedlicher Analyte, Parameter oder der gleichen eingesetzt werden. Insbesondere können die verschiedenen Träger 4 dann nacheinander in die Probe 2 bewegt bzw. mit dieser in Kontakt gebracht werden.

Je nach Bedarf können die Detektions- oder Bindungsbereiche 5 auf dem Träger 4 auch entfallen, insbesondere wenn der Träger 4 beispielsweise nur oder im Wesentlichen nur für Misch- oder Transportzwecke eingesetzt wird und/oder ein anderes Reaktionssystem oder mikrofluidisches System oder dergleichen bildet.

Weiter ist anzumerken, dass die Detektions- oder Bindungsbereiche 5 zwar besonders bevorzugt mikrostrukturiert sind, wie bereits erwähnt. Jedoch ist eine derartige Mikrostrukturierung nur optional.

Besonders bevorzugt erfolgt das Immobilisieren der Antikörper 7 auf dem Träger 4 bzw. dessen Oberfläche 4a bzw. in dem jeweiligen Detektions- oder Bindungsbereich 5 oder in verschiedenen Detektions- oder Bindungsbereichen 5 durch vorherige Plasmabeschichtung und/oder Silanisierung des Trägers 4, zumindest in den gewünschten Bereichen. Alternativ oder zusätzlich kann auch ein sogenanntes Fotolinking der Antikörper 7 erfolgen, wodurch die Dichte der gebundenen Antikörper 7 wesentliche erhöht werden kann. Grundsätzlich ist jedoch jede beliebige bzw. geeignete Behandlung möglich. Dies ist ein weiterer Vorteil der vorschlagsgemäßen Vorrichtung 1, des vorschlagsgemäßen Trägers 4 und des vorschlagsgemäßen Verfahrens.

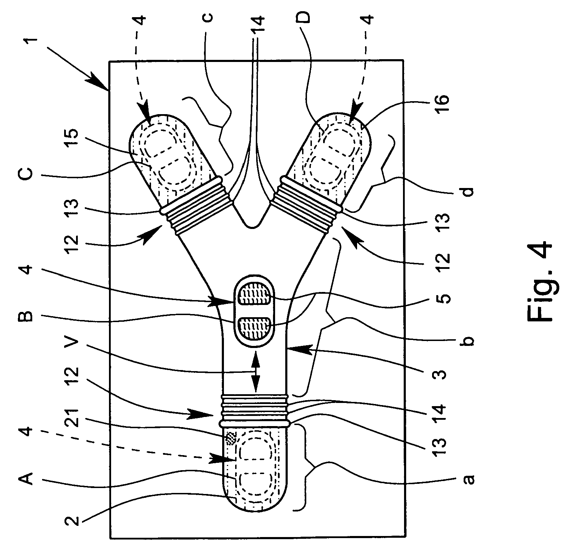

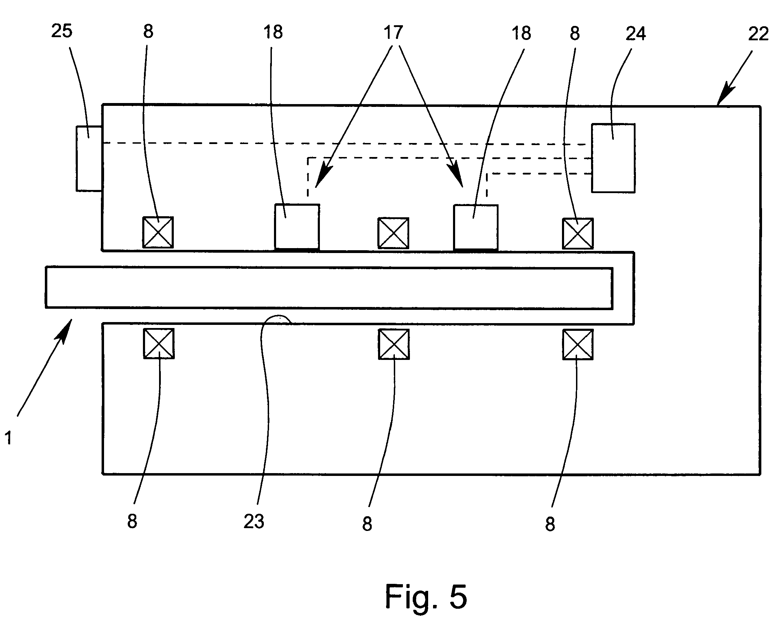

Nachfolgend weitere Ausführungsformen unter Bezugnahme auf Fig. 4 und 5 näher erläutert, wobei im Wesentlichen nur auf Unterschiede gegenüber der ersten Ausführungsform näher eingegangen wird. Die bisherigen Ausführungen und Erläuterungen gelten daher ergänzt oder entsprechend, auch wenn eine wiederholte Beschreibung weggelassen ist.

Fig. 4 zeigt in einer schematischen, zu Fig. 1 korrespondierenden Draufsicht eine zweite Ausführungsform der vorschlagsgemäßen Vorrichtung 1. Im Gegensatz zur ersten Ausführungsform weist der Kanal 3 hier eine Abzweigung auf oder bildet eine Art Weiche. Insbesondere ist hier der Träger 4 ausgehend von dem Bereich b bzw. von vorzugsweise nur einem einzigen Gasraum aus wahlweise in mehrere, insbesondere in mindestens drei verschiedene Bereiche a, c und d und/oder Reaktionskammern bewegbar, insbesondere wahlweise in mindestens drei verschiedene Flüssigkeiten, nämlich die Probe 2, die Flüssigkeit 15 und die Flüssigkeit 16.

Die Bewegung des Trägers 4 wird vorzugsweise wieder durch die hier nicht dargestellte Manipuliereinrichtung 8 gesteuert.

Die Vorrichtung 1 bzw. der Kanal 3 oder das vorzugsweise mikrofluidische System kann auch mehrere Verzweigungen oder eine Verzweigung mit noch mehr verschiedenen Kanalbereichen oder Kanalästen oder dergleichen aufweisen.

Bei Bedarf können auch mehrere Träger 4 in der Vorrichtung 1 bzw. dem Kanal 3 bzw. dem System eingesetzt werden, die je nach Anzahl der Abzweigungen, Reaktionskammern oder dergleichen in gewünschter Weise arrangiert werden können, insbesondere um gewünschte Verfahrensabläufe, Manipulationen und/oder Untersuchungen durchzuführen.

Fig. 5 zeigt in einem schematischen Schnitt eine vorschlagsgemäße Testeinrichtung 22 mit der vorschlagsgemäßen Vorrichtung 1. Die Testeinrichtung 22 weist insbesondere einen Aufnahmebereich 23 für die Vorrichtung 1 auf. Der Aufnahmebereich 23 ist insbesondere als Einführbereich und/oder schlitzartig ausgebildet. Jedoch ist es beispielsweise auch möglich, dass die Vorrichtung 1 zur Detektion bzw. Auswertung lediglich auf den Aufnahmebereich 23 der Testeinrichtung 22 aufgelegt wird oder in sonstiger Art und Weise mit der Testeinrichtung 22 bzw. dem Aufnahmebereich 23 in vorzugsweise mechanischen Kontakt oder zumindest in die Nähe davon gebracht wird, insbesondere um die magnetische Manipulation des Trägers 4 und/oder die vorzugsweise optische Detektion zu ermöglichen.

Die Testeinrichtung 22 weist vorzugsweise die Detektionseinrichtung 17 mit den Mess- bzw. Sensoreinrichtungen 18 zur bereits erläuterten Detektion, insbesondere optischen Detektion, besonders bevorzugt durch Lumineszenz und/oder Fluoreszenzmessung, auf.

Die Testeinrichtung 22 weist vorzugsweise die Manipuliereinrichtung 8 bzw. die hierzu vorzugsweise vorgesehenen Elektromagnete und besonders bevorzugt eine zugeordnete, nicht dargestellte Steuerung oder dergleichen auf. Mittels der Manipuliereinrichtung 8 wird der Träger 4 in gewünschter Weise wie bereits erläutert, in der Vorrichtung bzw. im Kanal 3 oder mikrofluidischen System bewegt.

Die Messwerte der Detektionseinrichtung 17 werden vorzugsweise von einer Auswerteeinrichtung 24 erfasst, aufgenommen, weiter ausgewertet, gespeichert und/oder weiterverarbeitet und beispielsweise über eine Ausgabeeinrichtung 25 der Testeinrichtung 22 ausgegeben. Bei der Ausgabeeinrichtung 25 kann es sich insbesondere um eine Anzeige, aber auch um eine Schnittstelle oder dergleichen handeln.

Die Testeinrichtung 22 stellt ein vorzugsweise mobiles Gerät dar, das zusammen mit der Vorrichtung 1 als POC-System einsetzbar ist. Das vorschlagsgemäße POC-System aus Testeinrichtung 22 und Vorrichtung 1 ist dementsprechend universell einsetzbar.

Die Vorrichtung 1 kann beispielsweise zunächst mit der Probe 2 befüllt werden und dann in den Aufnahmebereich 23 der Testeinrichtung 22 eingeführt werden, um die gewünschte Reaktion, Detektion und Auswertung vorzunehmen.

Es ist anzumerken, dass der Träger 4 in der Vorrichtung 1 vorzugsweise in einem vorbestimmten Bereich, beispielsweise dem Bereich a oder dem Bereich b sicher gehalten ist, bis die eigentliche Benutzung der Vorrichtung 1 erfolgt, insbesondere bis die Probe 2 eingefüllt ist. Hierzu kann die Vorrichtung 1 und/oder der Träger 4 mit einem entsprechenden Haltemittel versehen sein. Beim Haltemittel kann es sich beispielsweise um ein Anhaften, Ankleben oder dergleichen handeln. Das Haltemittel kann auch durch eine mechanische oder chemische Verbindung oder dergleichen gebildet sein. Das Haltemittel kann auch durch eine kraft- oder formschlüssige Verbindung, beispielsweise ein Einklemmen oder dergleichen, realisiert sein. Der Träger 4 kann auch magnetisch an einer gewünschten Position gehalten sein, beispielsweise durch Einbau eines Permanentmagneten in die Vorrichtung 1 bzw. das Basisteil 1A an einer gewünschten Stelle.

Einzelne Merkmale der verschiedenen Ausführungsformen und die Ausführungsformen selbst können beliebig kombiniert werden.

**Bezugszeichenliste**

| | | | |
|---|---|---|---|
| 1 | Vorrichtung | 18 | Mess- bzw. Sensoreinrichtung |
| 1A | Basisteil | 19 | Aufnahme |
| 1B | Deckel | 20 | Verbindungskanal |
| 1C | Entlüftungskanal | 21 | Reagenz |
| 2 | Probe | 22 | Testeinrichtung |
| 3 | Kanal | 23 | Aufnahmebereich |
| 4 | Träger | 24 | Auswerteeinrichtung |
| 4A | Oberfläche | 25 | Ausgabeeinrichtung |
| 5 | Detektions- bzw. Bindungsbereich | | |
| | | | a Bereich |
| 6 | Komplex | b | Bereich |
| 7 | Antikörper | c | Bereich |
| 8 | Manipuliereinrichtung | d | Bereich |
| 9 | Magnet | | e Bereich |
| 10 | Führungsmittel | | |
| 11 | Phasengrenze | A | Position |
| 12 | Mittel zum Zurückhalten von Flüssigkeit | B | Position |
| | | C | Position |
| 13 | Graben | D | Position |
| 14 | Nut | E | Position |
| 15 | Flüssigkeit | M | Magnetfeld |
| 16 | Flüssigkeit | V | Bewegungsrichtung |
| 17 | Detektionseinrichtung | | |

## Patentansprüche

1. Vorrichtung (1) zur Manipulation oder Untersuchung einer flüssigen Probe (2), mit einem mikrofluidischen Kanal (3) zur Aufnahme der Probe (2) und mit einem im Kanal (3) beweglichen Träger (4), der mindestens einen Detektions- oder Bindungsbereich (5) für einen Stoff aufweist, wobei der Stoff ein Analyt der Probe (2), ein davon gebildeter Komplex (6) oder davon abhängiges Reaktionsprodukt oder ein Reagenz, das mit einem Analyten der Probe (2), einem davon gebildeten Komplex (6) oder mit einem davon abhängigen Reaktionsprodukt wechselwirkt oder bindet, ist oder enthält,
**dadurch gekennzeichnet,**
**dass** die Vorrichtung (1) einen Gasraum aufweist und der Träger (4) in einer definierten Ausrichtung von dem Kanal (3) geführt und der Träger (4) eben oder plattenartig ausgebildet ist und der Träger (4) eine ebene Oberseite mit mehreren räumlich von einander getrennten Detektions- bzw. Bindungsbereichen (5) aufweist wobei der Träger (4) aus der Probe (2) heraus in den Gasraum zur Detektion des Stoffs bewegbar ist oder der Träger (4) durch den Gasraum in eine von der Probe (2) verschiedene Flüssigkeit oder umgekehrt bewegbar ist.

2. Vorrichtung nach Anspruch 1, **dadurch gekennzeichnet, dass** die Vorrichtung (1) zur optischen Detektion des Stoffs auf dem Träger (4) im Gasraum ausgebildet ist.

3. Vorrichtung nach einem der voranstehenden Ansprüche 1oder 2, **dadurch gekennzeichnet, dass** der Träger (4) eine Länge aufweist, die größer als der maximale Querschnitt des Kanals (3) ist.

4. Vorrichtung nach einem der voranstehenden Ansprüche, **dadurch gekennzeichnet, dass** der Träger (4) den Querschnitt des Kanals (3) zu mehr als 30% flächenmäßig ausfüllt.

5. Vorrichtung nach einem der voranstehenden Ansprüche, **dadurch gekennzeichnet, dass** der Träger (4) formstabil mit einer definierten Form ausgebildet ist.

6. Vorrichtung nach einem der voranstehenden Ansprüche, **dadurch gekennzeichnet, dass** der Träger (4) ein Kunststoffteil oder ein Spritzgussteil ist.

7. Vorrichtung nach einem der voranstehenden Ansprüche, **dadurch gekennzeichnet, dass** eine Manipuliereinrichtung (8) vorgesehen ist, um den Träger (4) aus der Probe (2) heraus zu bewegen.

8. Vorrichtung nach einem der voranstehenden Ansprüche, **dadurch gekennzeichnet, dass** der Detektions- oder Bindungsbereich (5) mikrostrukturiert ist.

9. Vorrichtung nach einem der voranstehenden Ansprüche, **dadurch gekennzeichnet, dass** der Träger (4) magnetisch und/oder berührungslos bewegbar ist.

10. Vorrichtung nach einem der voranstehenden Ansprüche, **dadurch gekennzeichnet, dass** der Träger (4) geradlinig in dem Kanal (3) bewegbar und/oder hin- und herbewegbar ist.

11. Vorrichtung nach einem der voranstehenden Ansprüche, **dadurch gekennzeichnet, dass** das Reagenz ein Antikörper (7) ist oder Antikörper (7) enthält.

12. Vorrichtung nach einem der voranstehenden Ansprüche, **dadurch gekennzeichnet, dass** der Detektions- oder Bindungsbereich (5) durch immobilisierte Antikörper (7) gebildet oder bedeckt ist.

13. Vorrichtung nach einem der voranstehenden Ansprüche, **dadurch gekennzeichnet, dass** die Vorrichtung (1) ein Immunoassay bildet.

14. Vorrichtung nach einem der voranstehenden Ansprüche, **dadurch gekennzeichnet, dass** die Vorrichtung (1) nur einen einzigen Träger (4) aufweist oder in dem Kanal (3) nur ein einziger beweglicher Träger (4) vorgesehen ist.

15. Vorrichtung nach einem der voranstehenden Ansprüche, **dadurch gekennzeichnet, dass** der Träger (4) länglich ausgebildet ist.

16. Vorrichtung nach einem der voranstehenden Ansprüche, **dadurch gekennzeichnet, dass** der Kanal (3) mindestens ein Mittel aufweist, um die Probe (2) in einem eine Reaktionskammer bildenden Bereich des Kanals (3) zu halten.

17. Vorrichtung nach einem der voranstehenden Ansprüche, **dadurch gekennzeichnet, dass** an einen eine Reaktionskammer bildenden Bereich des Kanals (3) mit der Probe (2) sich ein Gasraum des Kanals (3) anschließt und der Träger (4) über eine Phasengrenze hinweg und/oder in den Gasraum oder durch diesen hindurch bewegbar ist.

18. Vorrichtung nach einem der voranstehenden Ansprüche, **dadurch gekennzeichnet, dass** ein Binden des Stoffs oder sonstige Änderungen im Detektions- oder Bindungsbereich (5) zur Bestimmung eines Analyten in der Probe (2) - optisch detektierbar sind, wenn sich der Träger (4) in einem Gasraum befindet.

19. Vorrichtung nach einem der voranstehenden Ansprüche, **dadurch gekennzeichnet, dass** der Träger (4) wahlweise in verschiedene Bereiche des Kanals (3), in verschiedene Reaktionskammern und in mindestens eine von der Probe (2) verschiedene Flüssigkeit oder umgekehrt bewegbar ist.

## Claims

1. Device (1) for manipulating or analysing a liquid sample (2), comprising a microfluidic channel (3) for receiving the sample (2) and comprising a body (4) that is movable in the channel (3) and has at least one detection or binding region (5) for a substance, the substance being or containing an analyte of the sample (2), a complex (6) formed therefrom or reaction product dependent thereon, or a reagent that interacts or binds with an analyte of the sample (2), a complex (6) formed therefrom or a reaction product dependent thereon
**characterised in that**
the device (1) comprises a gas chamber and the body (4) is guided in a defined alignment by the channel (3) and the body (4) is planar or plate-like and the body (4) has a planar upper face which has a plurality of detection or binding regions (5) which are spatially separated from one another, it being possible to move the body (4) out of the sample (2) into the gas chamber for detecting the substance or it being possible to move the body (4) through the gas chamber into a liquid which is different from the sample (2), or vice versa.

2. Device according to claim 1, **characterised in that** the device (1) is designed for optical detection of the substance on the body (4) in the gas chamber.

3. Device according to either of the preceding claims 1 or 2, **characterised in that** the body (4) has a length that is greater than the maximum cross-section of the channel (3).

4. Device according to any of the preceding claims, **characterised in that** the body (4) fills the cross-section of the channel (3) by more than 30 % of its surface area.

5. Device according to any of the preceding claims, **characterised in that** the body (4) is dimensionally stable having a defined shape.

6. Device according to any of the preceding claims, **characterised in that** the body (4) is a plastics part of an injection-moulded part.

7. Device according to any of the preceding claims, **characterised in that** a manipulating device (8) is provided for moving the body (4) out of the sample (2).

8. Device according to any of the preceding claims, **characterised in that** the detection or binding region (5) is microstructured.

9. Device according to any of the preceding claims, **characterised in that** the body (4) is movable magnetically and/or in a contactless manner.

10. Device according to any of the preceding claims, **characterised in** the body (4) can be moved in a straight line in the channel (3) and/or can be moved back and forth.

11. Device according to any of the preceding claims, **characterised in that** the reagent is an antibody (7) or contains antibodies (7).

12. Device according to any of the preceding claims, **characterised in that** the detection or binding region (5) is formed or covered by immobilised antibodies (7).

13. Device according to any of the preceding claims, **characterised in that** the device (1) forms an immunoassay.

14. Device according to any of the preceding claims, **characterised in that** the device (1) comprises only one single body (4) or only one single movable body (4) is provided in the channel (3).

15. Device according to any of the preceding claims, **characterised in that** the body (4) is of elongate configuration.

16. Device according to any of the preceding claims, **characterised in that** the channel (3) comprises at least one means for holding the sample (2) in a region of the channel (3) that forms a reaction chamber.

17. Device according to any of the preceding claims, **characterised in that** a gas chamber of the channel (3) connects to a region of the channel (3) containing the sample (2) that forms a reaction chamber, and the body (4) can be moved away and/or into the gas chamber or through said gas chamber by means of a phase boundary.

18. Device according to any of the preceding claims, **characterised in that** binding of the substance or other changes in the detection or binding region (5) for determining an analyte in the sample (2) are optically detectable when the body (4) is in a gas chamber.

19. Device according to any of the preceding claims, **characterised in that** the body (4) can be moved selectively into various regions of the channel (3), into various reaction chambers and into at least one liquid that is different from the sample (2), or vice versa.

## Revendications

1. Dispositif (1) de manipulation ou d'analyse d'un échantillon (2) liquide, avec un canal (3) microfluidique pour la réception de l'échantillon (2) et un support (4) mobile dans le canal (3) qui présente au moins une zone de détection ou de liaison (5) pour une substance, dans lequel la substance est ou contient un analyte de l'échantillon (2), un complexe (6) formé de celui-ci ou un produit réactionnel dépendant de celui-ci ou un réactif qui interagit ou se lie avec un analyte de l'échantillon (2), un complexe (6) formé de celui-ci ou un produit réactionnel dépendant de celui-ci,
**caractérisé en ce que**
le dispositif (1) présente un espace de gaz et le support (4) est conduit dans une orientation définie du canal (3) et le support (4) est réalisé de façon plane ou de type plaque et le support (4) présente une face supérieure plane avec plusieurs zones de détection ou de liaison (5) spatialement espacées les unes des autres, dans lequel le support (4) peut être sorti de l'échantillon (2) dans l'espace de gaz pour la détection de la substance ou le support (4) peut être déplacé à travers l'espace de gaz dans un liquide différent de l'échantillon (2) ou inversement.

2. Dispositif selon la revendication 1, **caractérisé en ce que** le dispositif (1) est réalisé pour la détection optique de la substance sur le support (4) dans l'espace de gaz.

3. Dispositif selon l'une des revendications 1 ou 2 précédentes, **caractérisé en ce que** le support (4) présente une longueur qui est supérieure à la section transversale maximale du canal (3).

4. Dispositif selon l'une des revendications précédentes, **caractérisé en ce que** le support (4) remplit la section transversale du canal (3) à plus de 30 % en superficie.

5. Dispositif selon l'une des revendications précédentes, **caractérisé en ce que** le support (4) est réalisé de façon indéformable avec une forme définie.

6. Dispositif selon l'une des revendications précédentes, **caractérisé en ce que** le support (4) est une pièce en matière plastique ou une pièce moulée par injection.

7. Dispositif selon l'une des revendications précédentes, **caractérisé en ce qu'**un système de manipulation (8) est prévu pour sortir le support (4) de l'échantillon (2).

8. Dispositif selon l'une des revendications précédentes, **caractérisé en ce que** la zone de détection ou de liaison (5) est micro-structurée.

9. Dispositif selon l'une des revendications précédentes, **caractérisé en ce que** le support (4) peut être déplacé de façon magnétique et/ou sans contact.

10. Dispositif selon l'une des revendications précédentes, **caractérisé en ce que** le support (4) peut être déplacé en ligne droite dans le canal (3) et/ou en un mouvement de va-et-vient.

11. Dispositif selon l'une des revendications précédentes, **caractérisé en ce que** le réactif est un anticorps (7) ou contient un anticorps (7).

12. Dispositif selon l'une des revendications précédentes, **caractérisé en ce que** la zone de détection ou de liaison (5) est formée ou recouverte par des anticorps (7) immobilisés.

13. Dispositif selon l'une des revendications précédentes, **caractérisé en ce que** le dispositif (1) forme un essai immunologique.

14. Dispositif selon l'une des revendications précédentes, **caractérisé en ce que** le dispositif (1) présente uniquement un seul support (4) ou dans le canal (3) seulement un seul support (4) mobile est prévu.

15. Dispositif selon l'une des revendications précédentes, **caractérisé en ce que** le support (4) est réalisé longitudinalement.

16. Dispositif selon l'une des revendications précédentes, **caractérisé en ce que** le canal (3) présente au moins un moyen pour maintenir l'échantillon (2) dans une zone du canal (3) formant une chambre réactionnelle.

17. Dispositif selon l'une des revendications précédentes, **caractérisé en ce que**, au niveau d'une zone du canal (3) formant une chambre réactionnelle avec l'échantillon (2), s'adjoint un espace de gaz du canal (3) et le support (4) peut être avancé sur une limite de phase et/ou dans l'espace de gaz ou au travers de celui-ci.

18. Dispositif selon l'une des revendications précédentes, **caractérisé en ce qu'**une liaison de la substance ou d'autres modifications dans la zone de détection ou de liaison (5) peuvent être détectées optiquement pour la détermination d'un analyte dans l'échantillon (2), lorsque le support (4) se trouve dans un espace de gaz.

19. Dispositif selon l'une des revendications précédentes, **caractérisé en ce que** le support (4) peut être déplacé au choix dans différentes zones du canal (3), dans différentes chambres réactionnelles et dans au moins un liquide différent de l'échantillon (2) ou inversement.
